# EUROPEAN PATENT APPLICATION

(11) **EP 2 498 104 A2**
(43) Date of publication of application: **12.09.2012**
(21) Application number: 12001404.8
(22) Date of filing: 01.03.2012
(51) Int. Cl.: G01T 1/20

(54) **Radiation imaging device and method of manufacturing the same**

(30) Priority: 08.03.2011 JP 2011050154
(71) Applicant: Sony Corporation, Tokyo 108-0075 (JP)
(72) Inventor: Kusayama, Ikumi, Tokyo, 108-0075 (JP); Yokosawa, Nobuyuki, Tokyo, 108-0075 (JP); Kawanishi, Mitsuhiro, Tokyo, 108-0075 (JP); Igarashi, Takahiro, Tokyo, 108-0075 (JP)
(74) Representative: Müller - Hoffmann & Partner

(57) **Abstract**

A radiation imaging device includes: a sensor substrate having a pixel portion including a photoelectric conversion element; a scintillator layer provided on the pixel portion of the sensor substrate; and a sealing layer with which at least a part of the scintillator layer is sealed, in which the sealing layer includes a first wall portion disposed on the sensor substrate away from the scintillator layer, and a moisture-proof layer provided between the scintillator layer and the first wall portion.

## Description

### BACKGROUND

The present disclosure relates to a radiation imaging device which, for example, is suitable for X-ray photography for a medical care or a nondestructive inspection, and a method of manufacturing the same.

In recent years, various Flat Panel Detectors (FPDs) in which a large area and digitization are attained have been developed with the progress of a Thin Film Transistor (TFT) technique for liquid crystal display devices, and are used for a medical image analysis, an industrial nondestructive inspection or the like.

The FPD can acquire an image based on a radiation (such as an X-ray, an alpha-ray, a beta-ray, an electron ray or an ultraviolet) in the form of an electrical signal. As a result, a photographed image can be treated as digital information and, for example, storage, processing, transfer or the like of the photographed image is easy. The FPD includes two systems: (1) a direct conversion system for directly converting a radiation into an electrical signal; and (2) an indirect conversion system for reading out a visible light in the form of an electrical signal after a radiation has been temporarily converted into the visible light.

In particular, the FPD utilizing the indirect conversion system has a phosphor (scintillator) for absorbing a radiation and emitting a visible light on a substrate including a photoelectric conversion element. Some scintillators each of which has a deliquescence property and is easy to deteriorate due to moisture exist. For this reason, in the FPD utilizing the indirect conversion system, various devices are carried out in order to prevent the deterioration of the scintillator. Such techniques, for example, are disclosed in Japanese Patent No. 3077941 and JP-T-2002-518686 (hereinafter referred to as Patent Documents 1 and 2).

For example, Patent Document 1 proposes the technique for covering a surface of a scintillator with a protective film made of polyparaxylene (parylene C). The using of such a protective film makes it possible to protect the scintillator from the moisture.

### SUMMARY

However, with the technique disclosed in Patent Document 1, the protective film described above needs to be deposited by utilizing a Chemical Vapor Deposition (CVD) method. For this reason, a vacuum process needs to be used, and thus a deposition process is not simple. Then, Patent Document 2 proposes the technique for sticking a metallic plate onto a scintillator by using a sealing layer made of an adhesive agent, thereby sealing the scintillator with the metallic plate. With such a technique, the circumference (side portion) of the scintillator is sealed with the sealing layer made of the resin material.

With the technique disclosed in Patent Document 2, however, since the sealing layer is formed by utilizing an application method, an aspect ratio of the sealing layer depends on the property (so-called thixotropy or viscosity) of the material used. For this reason, in particular, when a thickness of the scintillator is large, a large installation space (application width) for the sealing layer needs to be ensured in the circumference of the scintillator. However, when there is a limit to a layout on a substrate, it may be impossible to sufficiently ensure the installation space for the sealing layer, and thus the sealing for the scintillator becomes insufficient. For this reason, in particular, when the thickness of the scintillator is large, the material having the high thixotropy or viscosity needs to be used as the sealing material, and thus usable materials are limited. Thus, there is desired the realization of a technique with which the scintillator can be sealed independently of the sealing material and the layout on the substrate.

The present disclosure has been made in order to solve the problems described above, and it is therefore desirable to provide a radiation imaging device in which the degree of freedom of material selection and a design can be increased in a sealing layer with which the circumstance of a scintillator layer is sealed, and a method of manufacturing the same.

In order to attain the desire described above, according to an embodiment of the present disclosure, there is provided a radiation imaging device including: a sensor substrate having a pixel portion including a photoelectric conversion element; a scintillator layer provided on the pixel portion of the sensor substrate; and a sealing layer with which at least a part of the scintillator layer is sealed. The sealing layer includes: a first wall portion disposed on the sensor substrate away from the scintillator layer; and a moisture-proof layer provided between the scintillator layer and the first wall portion.

The radiation imaging device according to the embodiment of the present disclosure has the first wall portion disposed away from the scintillator layer and the moisture-proof layer provided between the first wall portion and the scintillator layer as the sealing layer with which the scintillator layer is sealed in the circumference area of the pixel portion on the sensor substrate. Thus, the shape (the thickness and the width) of the moisture-proof layer (sealing layer) is controlled in accordance with the height and disposition portion of the first wall portion.

According to another embodiment of the present disclosure, there is provided a method of manufacturing a radiation imaging device including: forming a scintillator layer on a pixel portion of a sensor substrate having the pixel portion including a photoelectric conversion element; and forming a sealing layer in a circumference area of the pixel portion on the sensor substrate. In forming the sealing layer, after a first wall portion is formed in the circumference area, a moisture-proof layer is formed in an area on the pixel portion side of the first wall portion.

In the method of manufacturing a radiation imaging device according to another embodiment of the present disclosure, for formation of the sealing layer with which the scintillator layer is sealed in the circumference area of the pixel portion on the sensor substrate, after the first wall portion is formed away from the scintillator layer, the moisture-proof layer is formed between the first wall portion and the scintillator layer. Both of the height and disposition portion of the first wall portion are suitably set, whereby the shape (thickness and width) of the moisture-proof layer (sealing layer) is controlled.

As set forth hereinabove, according to the present disclosure, the first wall portion is provided disposed away from the scintillator layer and the moisture-proof layer is provided between the first wall portion and the scintillator layer as the sealing layer with which the scintillator layer is sealed in the circumference area of the pixel portion on the sensor substrate. Therefore, the shape of the moisture-proof layer (sealing layer) can be designed in accordance with the height and the like of the first wall portion. That is to say, the sealing layer can be formed at the desired aspect ratio independently of the material used in the moisture-proof layer. Therefore, the degree of freedom of the material selection and the design can be increased in the sealing layer with which the circumference of the scintillator layer is sealed.

### BRIEF DESCRIPTION OF THE DRAWINGS

FIG. 1 is a schematic cross sectional view showing a cross-sectional structure of a radiation imaging device according to a first embodiment of the present disclosure;
FIG. 2 is a schematic top plan view showing configurations of a pixel portion and a peripheral circuit thereof in the radiation imaging device according to the first embodiment of the present disclosure;
FIG. 3 is a circuit diagram showing a configuration of a pixel circuit (utilizing an active drive system) in a unit pixel shown in FIG. 2;
FIG. 4 is a circuit diagram showing a configuration of another pixel circuit (utilizing a passive drive system);
FIG. 5 is a cross sectional view showing a schematic structure of a sensor substrate shown in FIG. 1;
FIGS. 6A and 6B are respectively a top plan view showing a layout of a scintillator layer and a sealing layer disposed on a sensor substrate in the radiation imaging device shown in FIG. 1, and a partial cross sectional view of a structure in the vicinity of a boundary between a pixel portion and a peripheral portion thereof along line I - I of FIG. 6A;
FIGS. 7A, 7C, 7E, 7G, and FIGS. 7B, 7D, 7F, 7H are respectively top plan views explaining a method of manufacturing the radiation imaging device shown in FIG. 1 in the order of processes, and partial cross sectional views of structures in the vicinity of a boundary between a pixel portion and a peripheral portion thereof along line I - I of FIGS. 7A, 7C, 7E, and 7G;
FIGS. 8A and 8B are respectively a graph explaining a relationship between a glass plate thickness and an X-ray absorption rate, and a graph explaining a relationship between a bending stress applied to a glass plate, and a change in height;
FIG. 9 is a graph explaining a relationship between a curvature radius and a bending stress with a glass plate thickness as a parameter;
FIG. 10 is a partial cross sectional view showing a structure in the vicinity of a boundary between a pixel portion and a peripheral portion thereof in a radiation imaging device according to a second embodiment of the present disclosure;
FIGS. 11A, 11B, and 11C are respectively partial cross sectional views explaining a method of manufacturing the radiation imaging device shown in FIG. 10 according to the second embodiment of the present disclosure;
FIGS. 12A and 12B are respectively a top plan view showing a layout of a scintillator layer and a sealing layer disposed on a sensor substrate in a radiation imaging device according to Change 1 of the first embodiment, and a partial cross sectional view of a structure in the vicinity of a boundary between a pixel portion and a peripheral portion thereof along line I - I of FIG. 12A;
FIG. 13 is a partial cross sectional view showing a structure in the vicinity of a boundary between a pixel portion and a peripheral portion thereof in a radiation imaging device according to Change 2 of the first embodiment of the present disclosure;
FIG. 14 is a partial cross sectional view showing a structure in the vicinity of a boundary between a pixel portion and a peripheral portion thereof in a radiation imaging device according to Change 3 of the first embodiment of the present disclosure;
FIG. 15 is a partial cross sectional view showing a structure in the vicinity of a boundary between a pixel portion and a peripheral portion thereof in a radiation imaging device according to Change 4 of the first embodiment of the present disclosure;
FIG. 16 is a partial cross sectional view showing a structure in the vicinity of a boundary between a pixel portion and a peripheral portion thereof in a radiation imaging device according to Change 5 of the first embodiment of the present disclosure; and
FIG. 17 is a block diagram showing an entire configuration of a radiation imaging display system of an example of application to which the radiation imaging device according to the first embodiment of the present disclosure is applied.

### DETAILED DESCRIPTION OF THE PREFERRED EMBODIMENTS

Embodiments of the present disclosure will be described in detail hereinafter with reference to the accompanying drawings. It is noted that the description will be given below in accordance with the following order:
1. First Embodiment (the case where a thin plate glass is provided on a scintillator layer, and the circumference of the scintillator layer is sealed with a sealing layer having a moisture-proof layer provided between two wall portions);
2. Second Embodiment (the case where a wall portion is formed by laminating resin materials one upon another in a stepwise fashion);
3. Change 1 (the case where a sealing layer is composed of one wall portion and a moisture-proof layer);
4. Change 2 (the case where a moisture-proof layer is provided not only in the circumference of a scintillator layer, but also on an upper surface of the scintillator layer);
5. Change 3 (the case where a wall portion is not provided, but a thin plate glass is adhered onto a scintillator layer);
6. Change 4 (the case where a thin plate metal is provided on a scintillator layer);
7. Change 5 (the case where an upper surface of a scintillator layer is sealed with only a moisture-proof layer); and
8. Example of Application (an example of application of a radiation imaging display system).

### 1. First Embodiment

### [Structure and Configuration of Radiation Imaging Device]

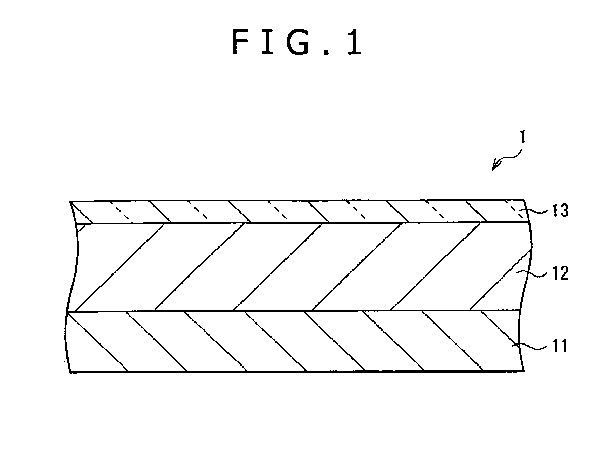

FIG. 1 shows a cross-sectional structure of a radiation imaging device (a radiation imaging device 1) according to a first embodiment of the present disclosure. The radiation imaging device 1 wavelength-converts a radiation typified by an alpha-ray, a beta-ray, a gamma-ray, or an X-ray, receives the resulting radiation, and reads out image information based on the radiation in the form of an electrical signal. Thus, the radiation imaging device 1 is suitably used as an X-ray imaging device for other nondestructive inspections such as a baggage inspection, including a use application for a medical care. The radiation imaging device 1 includes a scintillator layer 12 (scintillator layer) on a sensor substrate 11. Also, a thin plate glass 13 serving as a sealing plate is stuck onto an upper surface of the scintillator layer 12. Although details will be described later, in the first embodiment, the circumference (side surface) of the scintillator layer 12 is sealed with a sealing layer 14 (composed of a moisture-proof layer 14a, and wall portions 15A and 15B) which will be described later and which functions as an adhesion layer of the thin plate glass 13. Hereinafter, structures and configurations will be described in detail.

### (Sensor Substrate 11)

The sensor substrate 11 includes a pixel portion (a pixel portion 10A which will be described later), for example, composed of plural pixels on a substrate. Also, a drive circuit for driving the pixel portion 10A is disposed in a peripheral area (a peripheral area 10B) of the pixel portion 10A. The pixel portion 10A includes switch elements (transistors 111B, Tr1 to Tr3 which will be described later) each composed of a Thin Film Transistor (TFT), and a photoelectric conversion element (photodiodes 111A and PD which will be described later) every pixel. A thickness of such a sensor substrate 11 is preferably in the range of 50 to 700 µm from a viewpoint of durability and weight saving. Details (a pixel circuit and a cross-sectional structure) of the pixel portion 10A, and a configuration of a peripheral circuit (pixel drive circuit) will be described later.

### (Scintillator Layer 12)

The scintillator layer 12 is a layer made of a radiation phosphor which emits a fluorescence by irradiation of a radiation. A phosphor material is preferably one which absorbs an energy of a radiation and has a high efficiency of conversion from the radiation thus absorbed into an electromagnetic wave (an electromagnetic wave (light) ranging from an ultraviolet light to an infrared light with a visible light as a center), for example, having a wavelength of 300 to 800 nm. In addition, the phosphor material is preferably one which is easy to form a columnar crystal structure by utilizing an evaporation method. The reason for this is because the forming of the columnar crystal structure results in that scattering of an emitted light can be suppressed by a light guide effect, and the scintillator layer 12 can be thickened, thereby obtaining a high image resolution. A phosphor material which, for example, uses CsI as a base compound, and Tl or Na as an activator for compensating for a luminous efficiency or the like is given as an example of such a phosphor material. In addition, a thickness of the scintillator layer 12, for example, is in the range of 100 to 1,000 µm.

It is noted that the phosphor material used in the scintillator layer 12 is by no means limited to CsI, Tl and the like described above. For example, an alkali metal halide system phosphor expressed by a basic composition formula (I): M^{I}X · M^{II}X'₂ · bM^{III}X"₃ may also be used. In this basic composition formula (I), M^{I} represents at least one kind of alkali metal which is selected from the group consisting of lithium (Li), Na, kalium (K), rubidium (Rb), and Cs. M^{II} represents at least one kind of alkaline earth metal or bivalent metal which is selected from the group consisting of beryllium (Be), magnesium (Mg), calcium (Ca), Strontium (Sr), barium (Ba), nickel (Ni), copper (Cu), zinc (Zn), and cadmium (Cd). M^{III} represents at least one kind of rare earth element or trivalent metal which is selected from the group consisting of scandium (Sc), yttrium (Y), lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), aluminum (Al), gallium (Ga), and indium (In). In addition, each of X, X', and X" represents at least one kind of halogen element which is selected from the group consisting of fluorine (F), chlorine (Cl), bromine (Br), and iodine (I). A represents at least one kind of rare earth element or metal which is selected from the group consisting of Y, Ce, Pr, Nd, Sm, Eu, Gd, Tb, Dy, Ho, Er, Tm, Yb, Lu, Na, Mg, Cu, silver (Ag), Tl, and bismuth (Bi). In addition, a, b, and z represent values in the range of 0 ≤ a < 0.5, 0 ≤ b < 0.5, and 0 < z < 1.0, respectively. Moreover, M^{I} in the basic composition formula (I) preferably contains therein at least Cs, and X preferably contains therein at least I. In addition, A is preferably, especially Tl or Na, and z is preferably in the range of 1 × 10⁻⁴ ≤ z ≤ 0.1.

Also, in addition to the basic composition formula (I) described above, a rare earth-activated alkaline earth metal fluoride halide system phosphor expressed by a basic composition formula (II): M^{II}FX: zLn may also be used. In the basic composition formula (II) described above, M^{II} represents at least one kind of alkaline earth metal which is selected from the group consisting of Ba, Sr, and Ca. Ln represents at least one kind of rare earth element which is selected from the group consisting of Ce, Pr, Sm, Eu, Tb, Dy, Ho, Nd, Er, Tm, and Yb. X represents at least one kind of halogen element selected from the group consisting of Cl, Br, and I. In addition, z is in the range of 0 < z ≤ 0.2. It is noted that Ba preferably constitutes more than half of M^{II} in the basic composition formula (II) described above. Ln is preferably, especially Eu or Ce. In addition, a LnTaO₄: (Nb, Gd) system, a Ln₂SiO₅: Ce system, a LnOX: Tm system (Ln is a rare earth element), Gd₂O₂S: Tb, Gd₂O₂S: Pr, Ce, ZnWO₄, LnAlO₃: Ce, Gd₃Ga₂₅O₁₂: Cr, Ce, HfO₂, and the like are given in addition thereto.

### (Thin Plate Glass 13)

The thin plate glass 13, for example, is made of a glass having a thickness of 0.1 mm or less. The scintillator layer 12 is sealed from an upper surface thereof with the thin plate glass 13, and thus the thin plate glass 13 prevents the moisture from penetrating into the scintillator layer 12. As will be described later, the thin plate glass 13 is adhered to the sensor substrate 11 through the sealing layer 14, thereby sealing the scintillator layer 12 with the thin plate glass 13 together with the sealing layer 14. It is noted that resin coating for enhancing an impact resistance performance (for preventing the breaking due to micro-crack) may be made on a side surface of the thin plate glass 13, or another layer such as a reflecting film may be laminated between the thin plate glass 13 and the scintillator later 12. A sealing structure of the scintillator layer 12 will be described later.

### [Detailed Configuration of Sensor Substrate 11]

### (Pixel Portion and Peripheral Circuit)

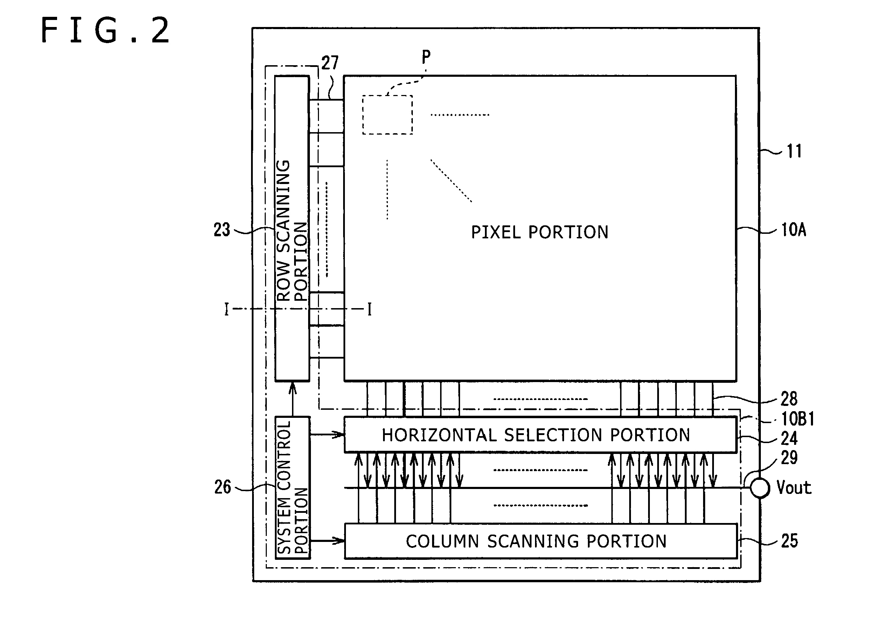

FIG. 2 schematically shows a configuration of a pixel portion 10A and a pixel drive circuit (a drive circuit utilizing an active matrix system) disposed in a peripheral area of the pixel portion 10A in the sensor substrate 11. In such a way, in the sensor substrate 11, a circuit portion 10B1 for driving the pixel portion 10A is disposed in the circumference of the pixel portion 10A. In the pixel portion 10A, pixels (unit pixels) P each including a photoelectric conversion element and transistors are two-dimensionally disposed in a matrix. The pixels P are connected to pixel drive lines 27 (specifically, row selection lines and reset control lines), and vertical signal lines 28.

The circuit portion 10B1, for example, includes a row scanning portion 23, a horizontal selection portion 24, a column scanning portion 25, and a system control portion 26. The row scanning portion 23 is configured so as to include a shift register, an address decoder, and the like. Thus, the row scanning portion 23 supplies drive signals to the pixel portion 10A through the pixel drive lines 27, thereby driving the pixel portion 10A in rows. The horizontal selection portion 24 is configured so as to include an amplifier, a horizontal selection switch, and the like which are provided every vertical signal line 28. The column scanning portion 25 is composed of a shift register, an address decoder, and the like, and drive horizontal selection switches of the horizontal selection portion 24 in order while it scans the horizontal selection switches of the horizontal selection portion 24. With such a configuration, in the circuit portion 10B1, electrical signals corresponding to quantities of received lights are successively read out from the pixels belonging to the pixel row subjected to the selective scanning (electrical signals are outputted to the vertical signal lines 28) to be transmitted to the outside through a horizontal signal line 29.

It is noted that a circuit portion composed of the row scanning portion 23, the horizontal selection portion 24, the column scanning portion 25, and the horizontal signal line 29 either may be a circuit configured through integration on the sensor substrate 11 or may be disposed in an external control IC connected to the sensor substrate 11. In addition, these circuit portions may also be formed on another substrate connected thereto through a cable or the like.

The system control portion 26 receives a clock supplied from the outside, data used to make an instruction for an operation mode, and the like, and outputs data such as internal information in the radiation imaging device 1. Also, the system control portion 26 includes a timing generator for generating various kinds of timing signals. Thus, the system control portion 26 carries out drive control for the row scanning portion 23, the horizontal selective portion 24, the column scanning portion 25, and the like in accordance with the various kinds of timing signals generated in the timing generator.

### (Pixel Circuit)

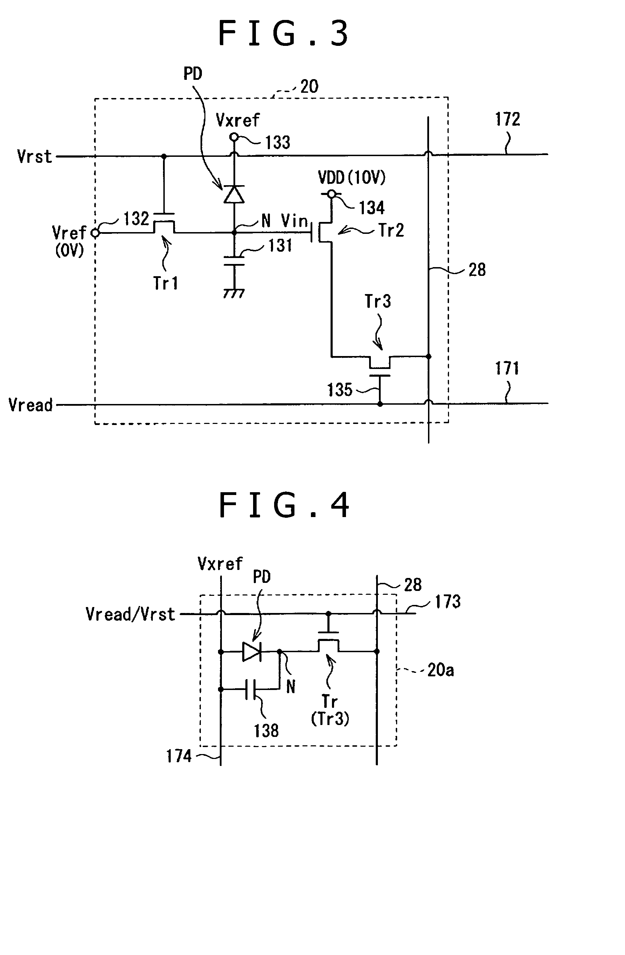

FIG. 3 shows a circuit (a pixel circuit 20) of the pixel P which is driven in accordance with the active matrix system as described above. The pixel circuit 20 is composed of a photodiode PD (a photoelectric conversion circuit), transistors Tr1, Tr2, and Tr3, the vertical signal line 28 described above, and the row selection line 171 and the reset control line 172 both of which serve as the pixel drive line 27.

The photodiode PD, for example, is a Positive Intrinsic Negative Diode (PIN) photodiode and, for example, a sensitivity region thereof (a wavelength region of a received light) becomes a visible region. A reference electric potential Vxref is applied to one terminal of the photodiode PD (a terminal 133, an upper electrode 125 which will be described later), whereby the photodiode PD generates signal electric charges having an amount of electric charges corresponding to a quantity of incident light (a quantity of received light). The other terminal (a p-type semiconductor layer 22 (lower electrode) which will be described later) of the photodiode PD is connected to a storage node N. A capacitance component 131 exists in the storage node N, and thus the signal electric charges generated in the photodiode PD are stored in the storage node N. It is noted that a configuration may also be adopted such that the photodiode PD is connected between the storage node N and the ground (GND).

Each of the transistors Tr1, Tr2, and Tr3, for example, is an N-channel field effect transistor. Thus, a silicon system semiconductor such as single crystal silicon, amorphous silicon, microcrystal silicon or polycrystal silicon, for example, is used in each of the transistors Tr1, Tr2, and Tr3. Or, a semiconductor oxide such as an indium gallium zinc oxide (InGaZnO) or a zinc oxide (ZnO) may also be used.

The transistor Tr1 is a reset transistor and is connected between a terminal 132 to which the reference electric potential Vref is applied, and the storage node N. The transistor Tr1 is turned ON in response to a reset signal Vrst, thereby resetting the electric potential at the storage node N to the reference electric potential Vref. The transistor Tr2 is a read transistor. A gate electrode of the transistor Tr2 is connected to the storage node N and a terminal 134 (a drain electrode thereof) is connected to the power source VDD. The transistor Tr2 receives the signal electric charges generated in the photodiode PD at a gate electrode thereof, and outputs a signal voltage corresponding to the signal electric charges thus generated. The transistor Tr3 is a row selection transistor and is connected between a source electrode of the transistor Tr2, and the vertical signal line 28. The transistor Tr3 is turned ON in response to a row scanning signal Vread to output the signal outputted from the transistor Tr2 to the vertical signal line 28. For the transistor Tr3, it is also possible to adopt a configuration such that the transistor Tr3 is connected between the drain electrode of the transistor Tr2 and the power source VDD.

It is noted that although in the case described above, the description has been given with respect to the circuit configurations of the pixel drive circuit utilizing the active drive system, and the pixel circuit, these circuit configurations may also be based on a passive drive system as will be described below. FIG. 4 shows a circuit configuration of a pixel circuit (a pixel circuit 20a) which is driven in accordance with the passive drive system. As shown in the figure, the pixel circuit 20a is composed of the photodiode PD, a capacitance component 138, and a transistor Tr (corresponding to the transistor Tr3 described above for read). The transistor Tr is connected between the storage node N and the vertical signal line 28. Thus, the transistor Tr is turned ON in response to the row scanning signal Vread, thereby outputting the signal electric charges stored in the storage node N based on the quantity of received light in the photodiode PD to the vertical signal line 28.

### (Cross-Sectional Structures of Photodiode and Transistor)

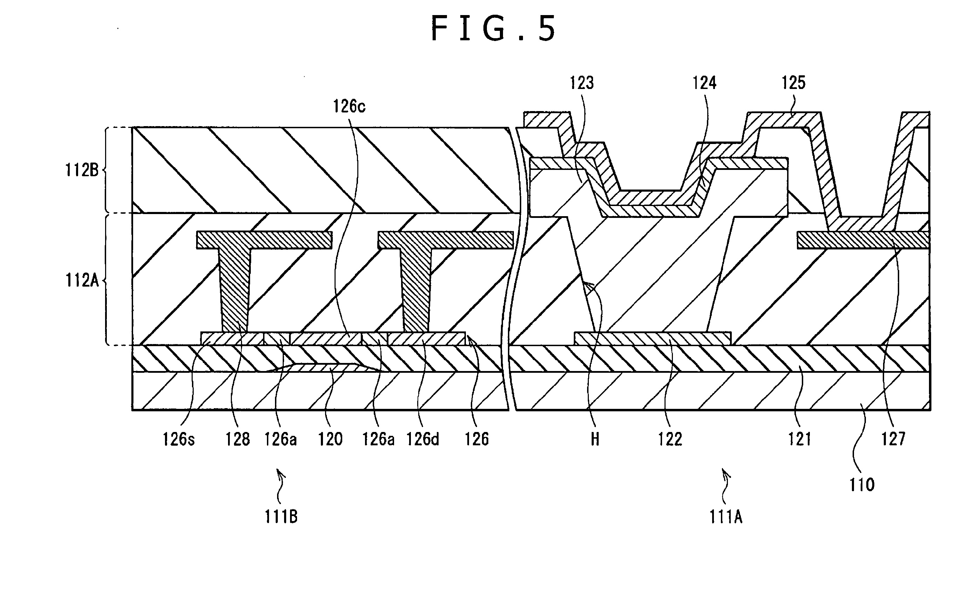

FIG. 5 shows a cross-sectional structure of each of the pixels P in the pixel portion 10A. In the pixel portion 10A, a photodiode 111A (corresponding to the photodiode PD), and a transistor 111B (corresponding to any one of the transistors Tr1 to Tr3) are provided every pixel P on a substrate 110.

In the photodiode 111A, a p-type semiconductor layer 122 is provided in a selective area on the substrate 110 made of a glass or the like through a gate insulating film 121. A first interlayer insulating film 112A having a contact hole (through hole) H is provided on the substrate 110 (specifically, on the gate insulating film 121) so as to face the p-type semiconductor layer 122. In the contact hole H of the first interlayer insulating film 112A, an i-type semiconductor layer 123 is provided on the p-type semiconductor layer 122, and an n-type semiconductor layer 124 is laminated on the i-type semiconductor layer 123. An upper electrode 125 is connected to the n-type semiconductor layer 124 through a contact hole provided in a second interlayer insulating film 112B. It is noted that although the case where the p-type semiconductor layer 122 and the n-type semiconductor layer 124 are provided on the substrate 110 side (lower side) and an upper side, respectively, has been given herein, a structure reverse to this structure, that is to say, a structure in which an n-type semiconductor layer and a p-type semiconductor layer are provided on the lower side and on the upper side, respectively, may also be adopted.

The gate insulating film 121, for example, becomes a layer common to the gate insulating film in the transistor 111B. Also, the gate insulating film 121, for example, is either a single-layer film made of any one of a silicon oxide, a silicon oxynitride, and a silicon nitride, or a lamination film obtained by laminating two or more kinds of materials of a silicon oxide, a silicon oxynitride, and a silicon nitride one upon another.

The p-type semiconductor layer 122 is a p⁺-type region obtained by, for example, doping either polycrystal silicon (polysilicon) or microcrystal silicon with boron (B). The p-type semiconductor layer 122, for example, serves as a lower electrode as well through which the signal electric charges are read out, and is connected to the storage node N described above (or the p-type semiconductor layer 122 becomes the storage node N in order to store therein the electric charges).

A first interlayer insulating film 112A, for example, is either a single layer film made of any one of a silicon oxide, a silicon oxynitride, and a silicon nitride, or a lamination film obtained by laminating two or more kinds of materials of a silicon oxide, a silicon oxynitride, and a silicon nitride one upon another. The first interlayer insulating film 112A, for example, is a layer common to an interlayer insulating film in the transistor 111B. A second interlayer insulating film 112B, for example, is made with a silicon oxide film.

The i-type layer semiconductor layer 123 is a semiconductor layer showing an intermediate conductive property between the p-type conductive property and the n-type conductive property, for example, a non-doped intrinsic semiconductor layer and, for example, is made of amorphous silicon. The n-type semiconductor layer 24, for example, is made of amorphous silicon and forms an n⁺-type region.

The upper electrode 125 is an electrode for supply of a reference electric potential for photoelectric conversion and, for example, is made with a transparent conductive film such as an Indium Tin Oxide (ITO). A power source wiring 127 for supply of a voltage to the upper electrode 125 is connected to the upper electrode 125. The power source wiring 127 is made of a material having a lower resistance than that of the upper electrode 125, for example, Ti, Al, Mo, W, Cr or the like.

In the transistor 111B, a gate electrode 120 made of Ti, Al, Mo, W, Cr or the like is formed on the substrate 110, and the gate insulating film 121 is formed on the gate electrode 120. A semiconductor layer 126 is provided in an area corresponding to the gate electrode 120 on the gate insulating film 121. For the purpose of reducing a leakage current, the semiconductor layer 126 includes a Lightly Doped Drain (LDD) 126a between a channel region 126c and a source region 126s, and the LDD 126a between the channel region 126c and a drain region 126d. Wiring layers 128 including a signal line for read and connected to the source region 126s and the drain region 126d, respectively, are provided on the semiconductor layer 126. The first interlayer insulating film 112A is provided so as to cover the wiring layers 128.

### [Peripheral Sealing Structure of Scintillator]

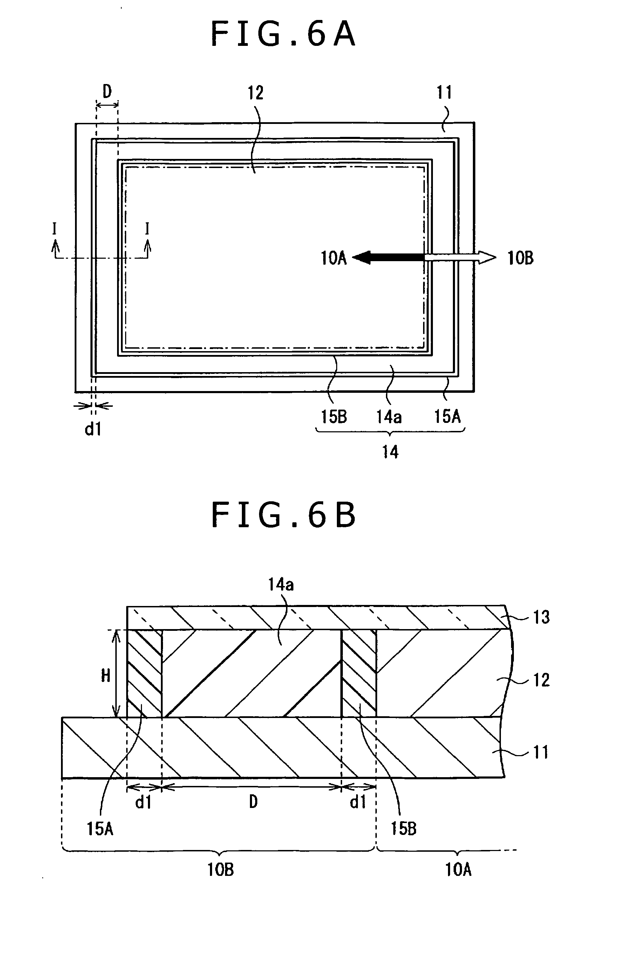

FIG. 6A is a top plan view showing a planar layout of the scintillator layer 12 and a sealing layer 114 which are disposed on the sensor substrate 11 as described above. FIG. 6B is a cross sectional view showing a cross-sectional structure in the vicinity of a boundary between the pixel portion 10A and a peripheral area 10B thereof. Also, FIG. 6B is a cross sectional view taken on line I - I of FIG. 6A. Thus, in FIG. 6B, an illustration of the thin plate glass 13 is omitted.

As has been described, in the first embodiment, the scintillator layer 12 is provided on the pixel portion 10A of the sensor substrate 11. Also, the sealing layer 14 with which the circumference of the scintillator layer 12 is sealed is formed in the peripheral area 10B of the pixel portion 10A. The thin plate glass 13 described above is disposed so as to cover the entire surfaces of the scintillator layer 12 and the sealing layer 14. Specifically, the thin plate glass 13 covers the pixel portion 10A and also extends from the pixel portion 10A to the peripheral area 10B thereof (the thin plate glass 13 has a substrate area slightly larger than that of the pixel portion 10A). Thus, the thin plate glass 13 is adhered to the sensor substrate 11 through the sealing layer 14 in the peripheral area 10B.

The circumference of the scintillator layer 12 is sealed with the sealing layer 14 in the manner as described above, and the sealing layer 14 functions as an adhesion layer through which the thin plate glass 13 is stuck to the scintillator layer 12. The sealing layer 14 is preferably formed so as to cover the entire side surface portion of the scintillator layer 12. The sealing layer 14 has a wall portion 15B, a moisture-proof layer 14a, and a wall portion 15A along the substrate surface in the order from the pixel portion 10A side (the scintillator layer 12 side), that is, holds the moisture-proof layer 14a between the two wall portions 15A and 15B.

The wall portions 15A and 15B have a function of controlling a width in the sealing layer 14 (the moisture-proof layer 14a) as well as a function as a spacer for controlling a thickness in the sealing layer 14 (the moisture-proof layer 14a). Each of the wall portions 15A and 15B has a height equal to the thickness of the scintillator layer 12, and is disposed so as to surround the scintillator layer 12 at a predetermined width, d1. The wall portions 15A and 15B are disposed at a predetermined interval (corresponding to a width D of the moisture-proof layer 14a) so as to become parallel with each other. In this case, in the plane along the substrate surface, the scintillator layer 12 is formed in a rectangular area corresponding to the pixel portion 10A. Also, each of the wall portions 15A and 15B is provided along four sides of the rectangular area (patterned into a frame-like shape). Each of the wall portions 15A and 15B, for example, is made of any one of an epoxy system resin (for example, a filler filled epoxy resin), an acylic system resin, a rubber-base silicon system resin, and a fluorine system resin. In addition, a width d1 of each of the wall portions 15A and 15B is preferably designed so as to have a size enough for the wall portions 15A and 15B themselves to be stably installed (without being bent, destroyed or position-shifted) between the sensor substrate 11 and the thin plate glass 13. The width d1, for example, is in the range of 0.3 to 10.0 mm. In addition, a height of each of the wall portions 15A and 15B is set in accordance with the thickness of the scintillator layer 12 and, for example, is in the range of 100 to 1,000 µm.

The moisture-proof layer 14a is made of a resin material which has a moisture-proof property (a property for preventing or suppressing the penetration of the moisture), and has an adhesion property for an adhesion receiving member (a glass in this case). In addition, the moisture-proof layer 14a is preferably made of the resin material whose change in the shape up to the adhesion curing is small. Any one of an epoxy system resin, for example, having a photo-curable property (UV curable property) or a thermosetting property (for example, a cresol novolak type, biphenyl type or brominated bisphenole type epoxy resin), an acylic system resin, polyurethane (PU), polystylene (PS), polyester (PE), and a fluorine system resin is used as such a resin material. Or, such a resin material may be composed of an adhesive agent made of an inorganic material like a so-called glass frit. A thickness H of the moisture-proof layer 14a, as described above, is controlled in accordance with the height of each of the wall portions 15A and 15B, and a width D thereof is controlled in accordance with the interval between the wall portions 15A and 15B. However, the width D of the moisture-proof layer 14a is suitably set from blend of the required moisture-proof property, and an installation area permitted on the sensor substrate 11 and, for example, is in the range of 3 to 30 mm.

In the first embodiment, in the moisture-proof layer 14a held between the wall portions 15A and 15B, as described above, the thickness H and the width D are arbitrarily set in accordance with the control for the height and interval of the wall portions 15A and 15B. Thus, the aspect ratio (H/D) of the thickness H to the width D can be freely designed. For example, when a limit or the like on the layout is taken into consideration, the aspect ratio (H/D), for example, is equal to or larger than 0.01. However, a high aspect ratio (equal to or larger than 0.6) which cannot be realized when the sealing is carried out by only using the moisture-proof layer (adhesion layer) without using any of the wall portions can also be freely designed.

It is noted that although the structure in which the wall portions 15A and 15B are formed in parallel with each other at the same width d1 has been exemplified herein, the widths of the wall portions 15A and 15B may be different from each other. In addition, although the interval (the width D) between the wall portions 15A and 15B is preferably, approximately constant in the circumference of the scintillator layer 12 from a viewpoint of carrying out the uniform hermetical sealing, the interval may differ in a part of an area surrounding the scintillator layer 12. For example, the circuit, the terminal for external connection, and the like are disposed in the peripheral area 10B. In this case, when there is a limit to the installation area and installation portion of the sealing layer 14 from the blend of the sealing layer 14, and the layout of the circuit, the terminal for external connection, and the like, the interval may be narrow (or wide) in a local portion.

### [Method of Manufacturing Radiation Imaging Device]

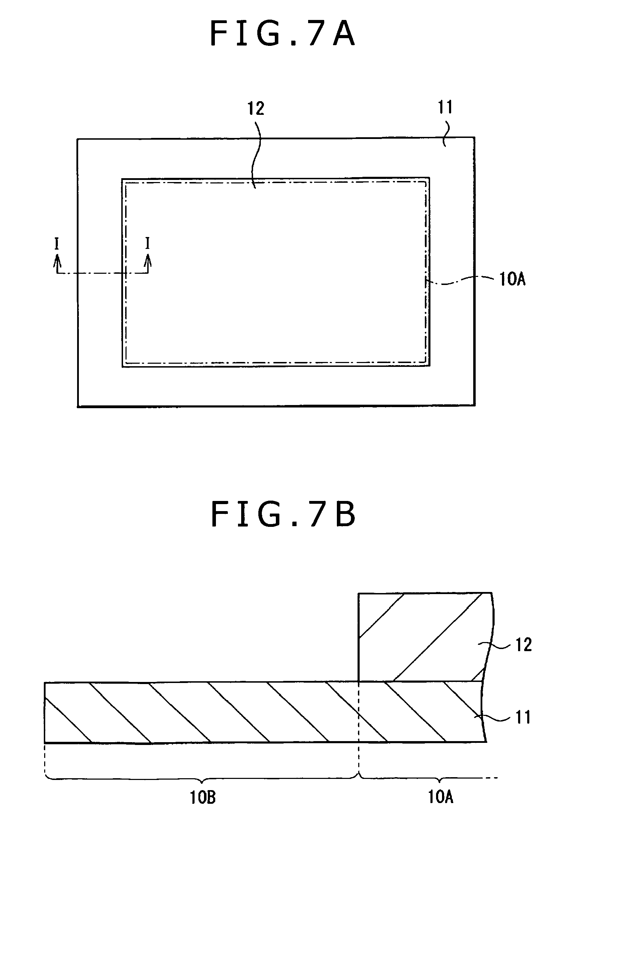

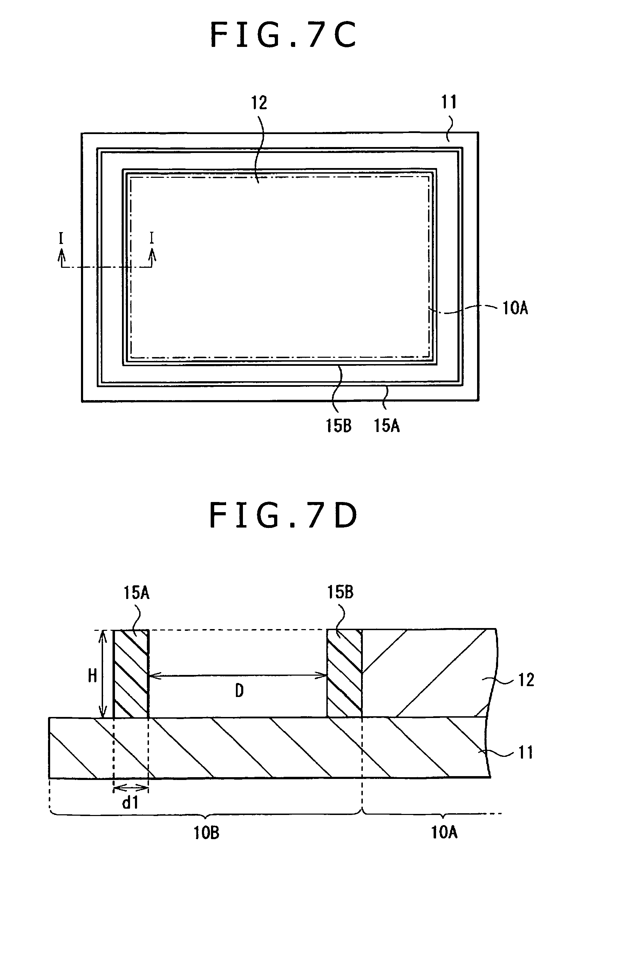

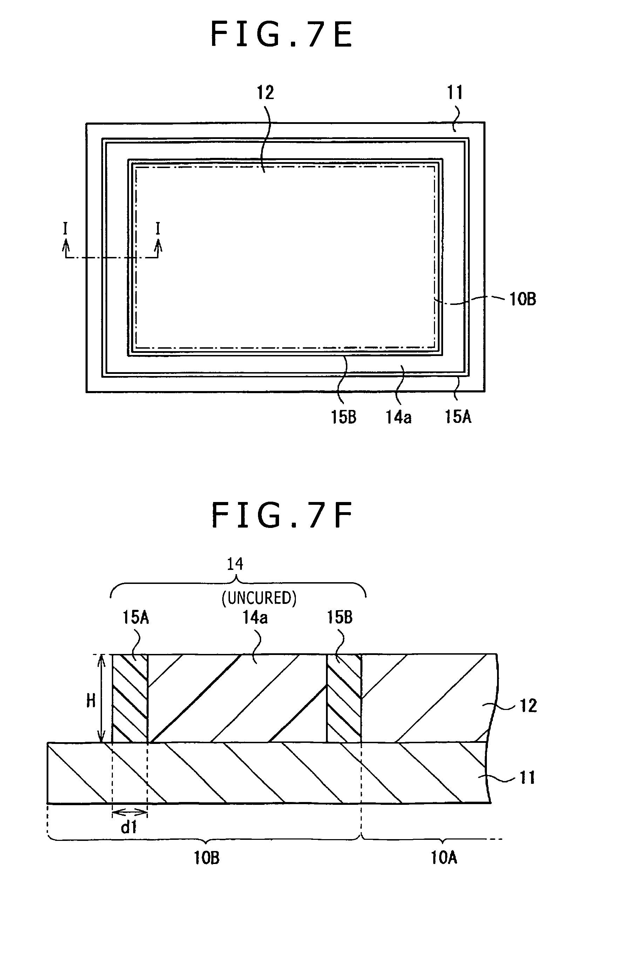

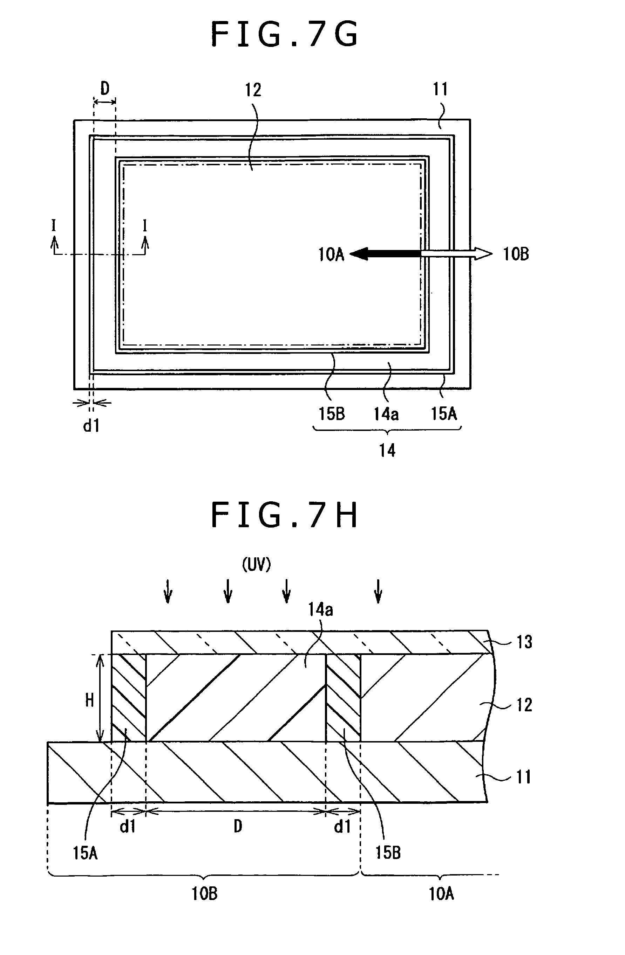

The radiation imaging device 1 as described above, for example, can be manufactured in the manner as will be described below. That is to say, firstly, the pixel portion 10A including the photodiode 111A and the transistor 111B, and the circuit portion 10B1 are formed on the substrate (the substrate 110 shown in FIG. 5), for example, made of a glass by utilizing the known thin film process, thereby making the sensor substrate 11. Hereinafter, processes for forming the scintillator layer 12 and the sealing layer 14 on the sensor substrate 11, and sticking the thin plate glass 13 onto the sensor substrate 11 will be described with reference to FIGS. 7A and 7B to FIGS. 7G and 7H. Note that, in FIGS. 7A and 7B to FIGS. 7G and 7H, FIGS. 7A, 7C, 7E, and 7G are respectively top plan views showing planar layouts of the scintillator layer 12 and the sealing layer 14 which are disposed on the sensor substrate 11 as described above. Also, FIGS. 7B, 7D, 7F, and 7H are respectively cross sectional views showing structures in the vicinity of a boundary between the pixel portion 10A and the peripheral area 10B thereof along lines I - I of FIGS. 7A, 7C, 7E, and 7G.

Firstly, as shown in FIGS. 7A and 7B, the scintillator layer 12 made of the material described above is disposed in an area corresponding to the pixel portion 10A of the sensor substrate 11 by, for example, utilizing a vacuum evaporation method. In this case, by using a mask having a rectangular opening corresponding to the pixel portion 10A, the scintillator layer 12 is formed only on the pixel portion 10A.

Subsequently, as shown in FIGS. 7C and 7D, both of the wall portions 15A and 15B are formed in the peripheral area 10B of the sensor substrate 11. Specifically, the silicon system material described above is applied by, for example, using a dispenser in accordance with the design as will be described below and is then cured, thereby forming both the wall portions 15A and 15B. In this case, the height (corresponding to the thickness H of the moisture-proof portion 14a which will be formed later) of each of the wall portions 15A and 15B, and the interval (corresponding to the width D of the moisture-proof portion 14a which will be formed later) between the wall portions 15A and 15B are designed so as to have the desired values, respectively. Specifically, the height of each of the wall portions 15A and 15B is set so as to become equal to the width of the scintillator layer 12 because the height of each of the wall portions 15A and 15B serves to control the thickness H of the moisture-proof layer 14a as described above. In addition, the interval between the wall portions 15A and 15B is set to a suitable value in consideration of the required moisture-proof property in the width direction, the limit on the layout of the sensor substrate 11, and the like because as described above, the interval between the wall portions 15A and 15B serves to determine the width D of the moisture-proof layer 14a. In addition, in this case, the wall portions 15A and 15B are respectively patterned into frame-like shapes so as to surround the circumference of the scintillator layer 12 at the predetermined width d1.

Next, as shown in FIGS. 7E and 7F, a resin material composing the moisture-proof layer 14a is applied and formed in the frame-like area surrounded by the two wall portions 15A and 15B (a resin material is poured into the frame-like area) by, for example, using the dispenser. In this case, an application amount of resin material is set to an amount with which the space defined between the wall portions 15A and 15B can be filled with the resin material composing the moisture-proof layer 14a up to the height equal to that of each of the wall portions 15A and 15B. In addition, although as described above, the epoxy system resin material, for example, is used in the moisture-proof layer 14a, not only the resin material having the high thixotropy and viscosity, but also the resin material having the low thixotropy and viscosity can also be used in the moisture-proof layer 14a. That is to say, the height and interval between the wall portions 15A and 15B are both controlled, whereby the thickness H and width D of the moisture-proof layer 14a are adjusted. Therefore, the moisture-proof layer 14a having an arbitrary aspect ratio can be formed without depending on the properties described above in the resin material used.

Next, as shown in FIGS. 7G and 7H, after the moisture-proof layer 14a is applied to the space defined between the two wall portions 15A and 15B in the manner described above, the thin plate glass 13 is piled on the scintillator layer 12 before the moisture-proof layer 14a is cured, and the moisture-proof layer 14a is then cured by, for example, carrying out the UV irradiation. In such a case, the sealing layer 14 is formed in the peripheral area 10B of the sensor substrate 11. As a result, in the peripheral area 10B of the sensor substrate 11, the circumference of the scintillator layer 12 is sealed with the sealing layer 14. Also, the thin plate glass 13 is stuck to the scintillator layer 12, and the scintillator layer 12 is hermetically sealed on the sensor substrate 11. With that, the radiation imaging device 1 shown in FIG. 1 and the like is completed.

It is noted that all it takes is that the order of formation of the scintillator layer 12, the moisture-proof layer 14a, and the wall portions 15A and 15B is the order which undergoes the process for forming the moisture-proof layer 14a after the process for forming the wall portions 15A and 15B, and thus is by no means limited to the order described above. For example, the moisture-proof layer 14a may be applied to the space defined between the two wall portions 15A and 15B after the wall portions 15A and 15B may be firstly formed in the peripheral area 10B of the sensor substrate 11, thereby forming the sealing layer 14, and finally, the scintillator layer 12 may be formed in the area surrounded by the sealing layer 14 by utilizing the evaporation method. Or, after firstly, the wall portions 15A and 15B may be formed, subsequently, the scintillator layer 12 may be formed and finally, the moisture-proof layer 14a may be applied to the space defined between the two wall portions 15A and 15B. In addition, the order of formation may also be made the order of formation of the wall portion 15B, the scintillator layer 12, the wall portion 15A, and the moisture-proof layer 14a. In such a way, it is only necessary to cause the formation order to undergo the process for forming the scintillator layer 12 after the process for forming the wall portion 15B. Note that, as a result, there is also obtained an effect that in the phase of deposition of the scintillator layer 12, it is suppressed that the phosphor material is adhered to the area in which the metallic wiring failure is feared in the peripheral circuit portion or the like.

### [Operation and Effects of Radiation Imaging Device]

### (Image Acquiring Operation)

Firstly, an imaging operation (image acquiring operation) of the radiation imaging device 1 will be described with reference to FIGS. 1 to 3, and FIG. 5. In the radiation imaging device 1, when the radiation which has been irradiated from a radiation (for example, an X-ray) irradiation source (not shown) to be transmitted through a subject (body to be detected) is made incident to the radiation imaging device 1, the radiation is subjected to the photoelectric conversion after having been subjected to the wavelength conversion, whereby an image of the subject is obtained in the form of an electrical signal. Specifically, after the radiation made incident to the radiation imaging device 1 has been transmitted through the thin plate glass 13, the radiation is absorbed in the scintillator layer 12. As a result, the scintillator layer 12, for example, emits a visible light which is in turn received in the pixel portion 10A (the pixels P) of the sensor substrate 11.

At this time, when a predetermined electric potential is supplied from the power source wiring (not shown) to the photodiode 111A through the upper electrode 125, the light, for example, made incident from the side of the upper electrode 125 is converted into the signal electric charges having the amount of electric charges corresponding to the quantity of received light (the photoelectric conversion is carried out). The signal electric charges generated through the photoelectric conversion are taken out in the form of a photocurrent from the side of the p-type semiconductor layer 122. Specifically, the electric charges generated through the photoelectric conversion in the photodiode 111A are collected by the storage layer (the p-type semiconductor layer 122, the storage node N), and are then read out in the form of a current from the storage layer to be supplied to the gate electrode of the transistor Tr2 (read transistor). Then, the transistor Tr2 outputs the signal voltage corresponding to the signal electric charges concerned. When the transistor Tr3 is turned ON in response to the row scanning signal Vread, the signal outputted from the transistor Tr2 is outputted (read out) to the vertical signal line 28. The signal thus read out is outputted to the outside through the horizontal selection portion 24. As a result, image data based on the radiation is obtained.

### (Operation by Sealing Layer 14)

In the radiation imaging device 1 as described above, as shown in FIGS. 6A and 6B, the sealing layer 14 is provided in the circumference (the peripheral area 10B) of the scintillator layer 12. The sealing layer 14 has the moisture-proof layer 14a for preventing (or suppressing) the penetration of the vapor. As a result, it is suppressed that the moisture penetrates from the side surface into the scintillator layer 12. In the sealing layer 14 in the first embodiment, such a moisture-proof layer 14a is held between the two wall portions 15A and 15B.

Here, the scintillator layer 12, for example, is formed on the sensor substrate 11 so as to have the thickness as described above. In this case, the sealing layer 14 is formed so as to cover the side surface portion of the scintillator layer 12, that is, the sealing layer 14 is provided in such a way that the thickness of the moisture-proof layer 14a becomes equal to that of the scintillator layer 12. As a result, the circumference of the scintillator layer 12 is hermetically sealed.

Now, the moisture-proof layer 14a is made of the resin material (such as the epoxy system resin) having the moisture-proof property as described above. In this case, the moisture-proof layer 14a is formed by directly applying such a resin material onto the peripheral area 10B on the sensor substrate 11. For this reason, if the moisture-proof layer 14a is singularly formed in the circumference of the scintillator layer 12 (without using any of the wall portions 15A and 15B), the aspect ratio (the width H/the thickness D) depends on the properties (the thixotropy and viscosity) of the resin material used. When the thickness of the scintillator layer 12 is large and thus there is a limit to the sealing layer installation space (application width) in the peripheral area 10B, the material having the high thixotropy and viscosity needs to be used as the sealing material, and thus the usable materials are limited.

On the other hand, the first embodiment adopts the structure in which the moisture-proof layer 14a is held between the two wall portions 15A and 15B. Specifically, in the process for depositing the sealing layer 14, after the two wall portions 15A and 15B have been installed in the peripheral area 10B so as to have the predetermined interval and height, the resin material is applied (poured) as the moisture-proof layer 14a to (into) the space defined between the two wall portions 15A and 15B. As a result, both of the wall portions 15A and 15B function as a dam, and thus the resin material thus applied is accumulated in the space defined between the two wall portions 15A and 15B. That is to say, the width D of the moisture-proof layer 14a is determined in accordance with the interval between the wall portions 15A and 15B, and the thickness H thereof is determined based on the height of each of the wall portions 15A and 15B. Therefore, the height and disposition portions of the wall portions 15A and 15B are suitably set in consideration of the thickness of the scintillator layer 12, and the required moisture-proof property, the installation space, and the like, whereby the shape (the width D and thickness H) of the moisture-proof layer 14a is controlled.

Therefore, even when, for example, the thickness of the scintillator layer 12 is large, the moisture-proof layer 14a can be designed based on the desired aspect ratio (H/D) without limiting the usable resin materials. Therefore, the degree of freedom of the material selection and the design is increased in the sealing layer 14 surrounding the scintillator layer 12. For example, even when the resin material having the low viscosity (for example, the epoxy system resin having the low thixotropy and viscosity in spite of the excellent moisture-proof property) is used in the moisture-proof layer 14a, it is possible to realize the high aspect ratio (H/D).

Here, TABLE 1 shows an aspect ratio when a high-viscosity material A (having a viscosity of 120 Pa ·s) and a low-viscosity material B (having a viscosity of 48 Pa · s) were each used as the moisture-proof resin, and the moisture-proof layer 14a was formed by using the two wall portions 15A and 15B (in the case of the first embodiment), and an aspect ratio when the moisture-proof layer 14a was formed without using any of the wall portions 15A and 15B. It is noted that the setting of the dispenser was made identical between the case of provision of the wall portions, and the case of no provision of the wall portions. As shown in TABLE 1, even when the moisture-proof resin was either the high-viscosity material A or the low-viscosity material B, the aspect ratio in the case of provision of the wall portions which was about three times as large as that in the case of no provision of the wall portions was obtained.

**TABLE 1**

| Moisture-proof resin | High-viscosity material A | | Low-viscosity material B | |
|---|---|---|---|---|
| Wall portions | Provision | No provision | Provision | No provision |
| Width (mm) | 1.71 | 3.04 | 2.87 | 6.51 |
| Thickness (mm) | 2.41 | 1.56 | 1.87 | 1.56 |
| Aspect ratio | 1.41 | 0.51 | 0.65 | 0.24 |

### (Operation by Thin Plate Glass 13)

In the first embodiment, the thin plate glass 13 is stuck onto the upper surface of the scintillator layer 12 (adhered to the sensor substrate 11 by the sealing later 14), and the scintillator layer 12 is hermetically sealed with the thin plate glass 13 and the sealing layer 14 described above. Thus, the using of the glass plate as a top plate for the scintillator layer 12 results in that the penetrating of the moisture into the scintillator layer 12 is suppressed and also the following merits are obtained.

That is to say, the using of the thin film plate 13 results in that the manufacturing processes are simplified as compared with the case where, for example, the sealing is carried out by using the organic protective film such as parylene C. Specifically, when the organic protective film is used, the film deposition process is not simple because the film deposition process requires the vacuum process such as the CVD method. However, the using of the glass plate results in that the scintillator layer 12 can be sealed without carrying out such a vacuum process.

In addition, since the glass is used as the base material even in the sensor substrate 11 becoming an object of the sticking of the thin plate glass 13, there is obtained the structure in which the two sheets of glasses are stuck to each other through both of the sealing layer 14 and the scintillator layer 12. For this reason, a difference in coefficient of linear expansion between the sensor substrate 11 and the thin plate glass 13 is hard to occur and thus the warpage of the panel due to the heat is difficult to generate.

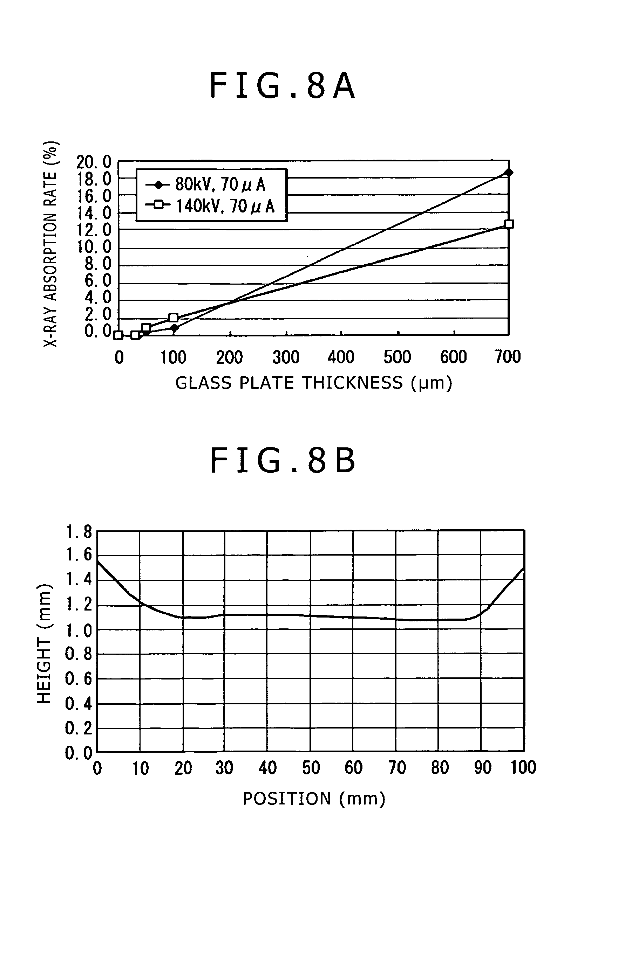

Moreover, in the thin plate glass 13, an X-ray absorption rate becomes low (an X-ray transmission rate becomes high) as the thickness of the thin plate glass 13 becomes smaller, while a moisture penetration probability does not depend on the thickness of the thin plate glass 13 (in the case of the glass, even when the thickness thereof is small, the penetration of the moisture can be sufficiently suppressed). Here, FIG. 8A shows a relationship (actual measured values) between a glass plate thickness (µm) and an X-ray absorption rate (%). In this case, the measurements were carried out under the condition in which tube voltages were set to 80 kV and 140 kV, respectively (a tube current was set to 70 μA in each of the cases). In such a way, it was confirmed that when the thickness of the thin plate glass was in the range of 0.03 to 0.1 mm (30 to 100 µm), the X-ray absorption rate becomes equal to or smaller than about 2.0%, and thus the excellent X-ray absorption rate can be realized.

In addition, FIG. 8B shows a height distribution of the thin plate glass 13 after the sealing layer 14 as described above was formed on a glass plate (supposed to be the sensor substrate 11) having a thickness of 0.7 mm, and a glass plate (the thin plate glass 13) having a thickness of 0.1 mm is stuck onto the sealing layer 14 through heat curing. As apparent from FIG. 8B, in the manufacturing processes, the sticking of the sensor substrate 11 and the thin plate glass 13 causes a stress to be generated in the panel and as a result, a curvature radius of a curved surface of the thin plate glass 13 in the adhesion surface to the sealing layer 14 reaches 60 to 80 mm.

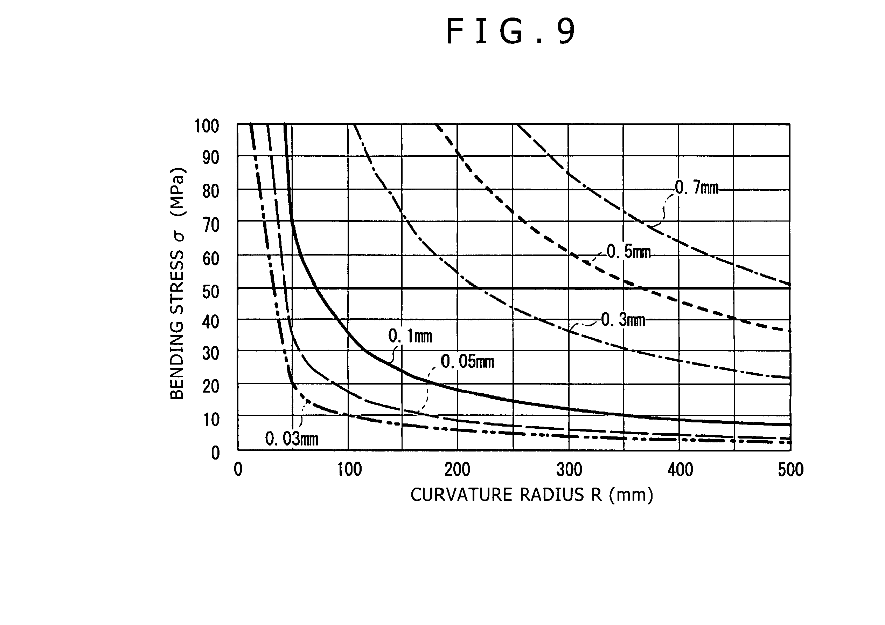

FIG. 9 shows a relationship between a curvature radius R (mm) in a phase of curving, and a bending stress σ (MPa) applied to a glass plate in the phase of the curving with respect to the cases where a glass plate thickness is set to 0.03 mm, 0.05 mm, 0.1 mm, 0.3 mm, 0.5 mm, and 0.7 mm. When the long term use of the glass is taken into consideration, the allowable bending stress is 50 MPa (the probability that the glass is destroyed by the stress of 50 MPa or more is increased). As described above, since the curvature radius of the glass which is bent in the manufacturing processes is in the range of 60 to 80 mm, the bending stress which is applied to the glass in that range of the curvature radius preferably becomes equal to or smaller than 50 MPa. It is understood from the graph shown in FIG. 9 that the glass thickness meeting this condition is equal to or smaller than 0.1 mm. That is to say, this means that when the glass thickness is set equal to or smaller than 0.1 mm, the glass plate has an ability (flexible property) to relax the bending stress generated in the manufacturing processes. In addition, when the manufacturing property is taken into consideration, a minimum value of the glass thickness is about 0.03 mm. In consideration of these results, the thickness of the thin plate glass is preferably in the range of 0.03 to 0.1 mm.

In addition thereto, as described above, while the thin plate glass 13 is stuck to the sensor substrate 11, a deformation (for example, a change in a thickness) following the curing is caused in the sealing layer 14 (specifically, the moisture-proof layer 14a) in some cases. The thin plate glass 13 having the thickness of 0.1 mm or less can follow such a deformation of the sealing layer 14 because the thin plate glass 13 has the flexibility described above.

As described above, in the first embodiment, for formation of the sealing layer 14 with which the circumference of the scintillator layer 12 is sealed on the sensor substrate 11, the wall portion 15A is provided away from the scintillator layer 12, the well portion 15B is further provided inside the well portion 15A, and the moisture-proof layer 14a is formed in the space defined between the two wall portions 15A and 15B. As a result, the shape (the width D and the thickness H) of the moisture-proof layer 14a can be designed in accordance with the height, the disposition interval, and the like of the wall portions 15A and 15B. That is to say, for example, even when the thickness of the scintillator layer 12 is large, the sealing layer 14 can be formed at the desired aspect ratio irrespective of the installable space in the peripheral area 10B of the sensor substrate 11, and irrespective of the material used in the moisture-proof layer 14a. Therefore, the degree of freedom of the material selection and the design can be increased in the sealing layer 14 with which the circumference of the scintillator 12 is sealed.

### 2. Second Embodiment

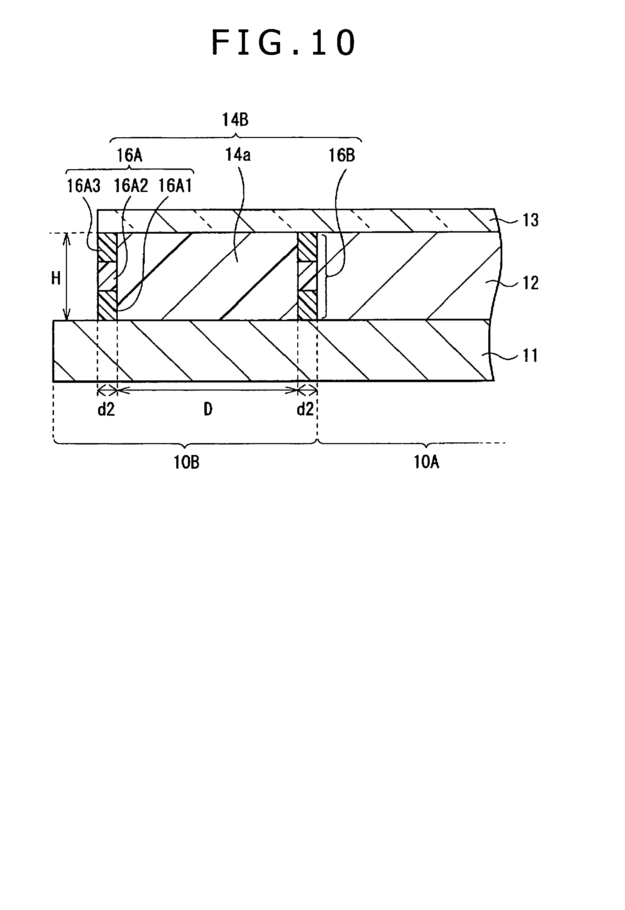

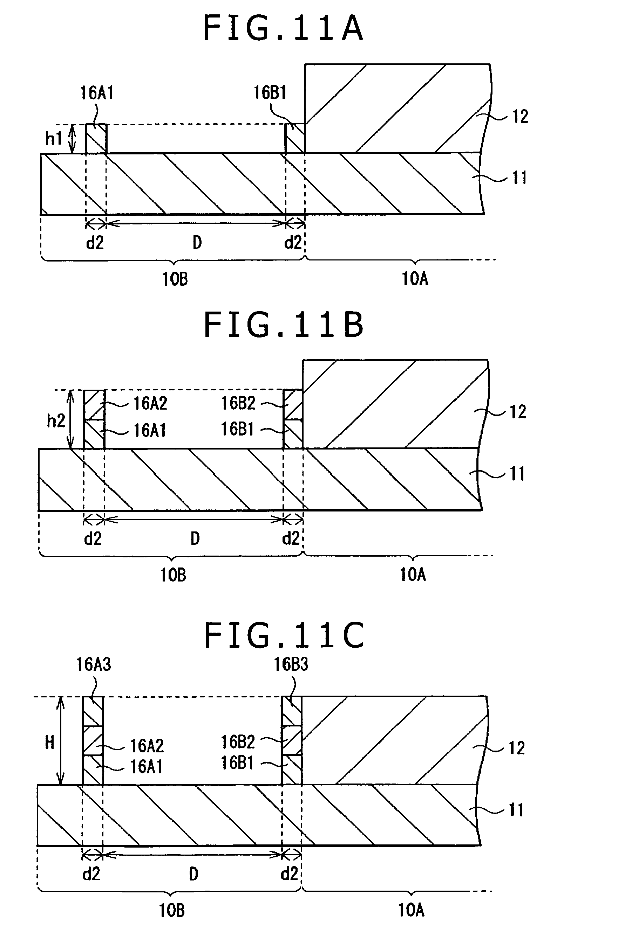

Next, a radiation imaging device according to a second embodiment of the present disclosure will be described in detail with reference to FIG. 10 and FIGS. 11A to 11C. It is noted that constituent elements similar to those in the radiation imaging device 1 according to the first embodiment of the present disclosure are designated by the same reference numerals or symbols, respectively, and a description thereof is omitted here for the sake of simplicity. In addition, in this case, for the sake of convenience, points (a sealing structure of the circumference of the scintillator layer, and a manufacturing method) different from the case of the radiation imaging device 1 of the first embodiment will be mainly described below.

### [Sealing Structure of Circumference of Scintillator Layer]

FIG. 10 is a schematic cross sectional view showing a cross-sectional structure of the vicinity of the boundary between the pixel 10A and the peripheral area 10B. In the second embodiment as well, similarly to the case of the first embodiment described above, a sealing layer 14B with which the circumference of the scintillator layer 12 is sealed functions as an adhesion layer for the thin plate glass 13, and the moisture-proof layer 14a is held between two wall portions 16A and 16B. However, in the second embodiment, in the sealing layer 14B, each of the wall portions 16A and 16B is applied and formed in a multi-stepwise fashion, that is, formed by lamination plural resin layers one upon another. In this case, the wall portions 16A and 16B have three resin layers 16A1 to 16A3, and 16B1 to 16B3, respectively.

The wall portions 16A and 16B serve to control the thickness H and width D of the moisture-proof layer 14a similarly to the case of the wall portions 15A and 15B in the first embodiment described above. In addition, the wall portions 16A and 16B surround the scintillator layer 12 and are disposed at a predetermined interval (corresponding to the width D of the moisture-proof layer 14a) in parallel with each other. Although the wall portions 16A and 16B are made of the same resin materials as those of the wall portions 15A and 15B in the first embodiment described above, the resin layers 16A1 to 16A3, and 16B1 to 16B3 either may be made of the same material or may be made of materials different from one another.

A height of each of the wall portions 16A and 16B is set in accordance with the thickness of the scintillator layer 12 similarly to the case of the first embodiment described above. However, a width d2 of each of the wall portions 16A and 16B, for example, is set in the range of 0.3 to 10.0 mm, and thus may be set narrower than that of each of the wall portions 15A and 15B in the first embodiment described above.

### [Manufacturing Method]

The radiation imaging device having the sealing layer 14B as described above, for example, can be manufactured in the manner which will be described below. That is to say, firstly, after the sensor substrate 11 is made similarly to the case of the first embodiment described above, the scintillator layer 12 is formed on the sensor substrate 11. After that, the sealing layer 14B as described above is formed. A process for forming the wall portions 16A and 16B in the sealing layer 14B will be described below with reference to FIGS. 11A to 11C.

Firstly, as shown in FIG. 11A, the wall portions 16A and 16B are formed in the peripheral area 10B of the sensor substrate 11. Specifically, the resin material as described above is applied so as to have a height h1 and a width d2, thereby forming the resin layers 16A1 and 16B1. Subsequently, as shown in FIG. 11B, the resin material identical to or different from that resin material is applied onto the resin layers 16A1 and 16B1 thus formed by, for example, using the dispenser so as to have the same width (the width d2) as that of each of the wall portions 16A1 and 16B1, and so as to have a height h2 in total, thereby forming the resin layers 16A2 and 16B2. Subsequently, as shown in FIG. 11C, the resin material identical to or different from each of the resin materials of the resin layers 16A2 and 16B2 is applied onto the resin layers 16A2 and 16B2 by, for example, using the dispenser so as to have the same width (the width d2) as that of each of the wall portions 16A1, 16A2, and 16B1, 16B2 and so as to have the height in total which is equal to the thickness (the thickness H of the moisture-proof layer 14a) of the scintillator layer 12. The resin layers 16A3 and 16B3 are deposited in such a manner, thereby forming the wall portions 16A and 16B in a multi-stepwise fashion.

After that, similarly to the case of the first embodiment described above, the resin material becoming the moisture-proof layer 14a is poured into the space defined between the two wall portions 16A and 16B. Also, the thin plate glass 13 is piled on the sensor substrate 11 through the scintillator layer 12 and the sealing layer 14B, and the resin material is then cured by, for example, carrying out the UV irradiation, thereby hermetically sealing the scintillator layer 12.

Note that, in the second embodiment as well, similarly to the case of the first embodiment described above, all it takes is that the order of formation of the scintillator layer 12, the wall portions 16A and 16B, and the moisture-proof layer 14a is made the order which undergoes the process for forming the moisture-proof layer 14a after the process for forming the wall portions 16A and 16B, and thus is by no means limited to the order of formation described above. For example, after the sealing layer 14B may be formed in the peripheral area 10B of the sensor substrate 11, the scintillator layer 12 may be deposited in the area surrounded by the sealing layer 14B by utilizing the evaporation method. Or, the order of formation may also be made the order of formation of the wall portions 16A and 16B, the scintillator later 12, and the moisture-proof layer 14a. In addition, the order of formation may also be made the order of formation of the wall portion 16B, the scintillator layer 12, the wall portion 16A, and the moisture-proof layer 14a.

### [Operation and Effects]

In the radiation imaging device as well of the second embodiment, similarly to the case of the first embodiment described above, when the radiation is absorbed in the scintillator layer 12 after having been transmitted through the thin plate glass 13, the visible light is emitted from the scintillator layer 12 and is then received by the pixel portion 10A (the pixels P) of the sensor substrate 11. The electrical signals corresponding to the quantities of received lights are read out from the pixels P, respectively, thereby obtaining the pixel data based on the radiation.

In addition, in the sealing layer 14B with which the circumference of the scintillator layer 12 is sealed, the moisture-proof layer 14a is held between the two wall portions 16A and 16B, whereby the resin material applied to the space defined between the two wall portions 16A and 16B in the process for depositing the moisture-proof layer 14a is accumulated in the space defined between the two wall portions 16A and 16B. That is to say, the width D and thickness H of the moisture-proof layer 14a are controlled in accordance with the interval between the wall portions 16A and 16B, and the height of each of the wall portions 16A and 16B. Therefore, similarly to each of the first embodiment described above, even when, for example, the thickness of the scintillator layer 12 is large, the moisture-proof layer 14a can be designed based on the desired aspect ratio (H/D) without limiting the usable resin materials. Therefore, it is possible to obtain the same effects as those of the first embodiment described above.

However, in the second embodiment, the wall portions 16A and 16B are formed in the form of the multilayer structures (applied and formed in the multi-stepwise fashion) in the sealing layer 14B, whereby the width d2 of each of the wall portions 16A and 16B can be more finely (finely without changing the height), and stably formed. That is to say, the width d2 of each of the wall portions 16A and 16B is preferably designed so as to have a size with which the wall portions 16A and 16B themselves are stably installed similarly to the case of the width d1 of each of the wall portions 15A and 15B described above. However, the adopting of the multilayer structure as described above results in that even when the width, d2, is made fine, the sufficient stability can be held. For this reason, this structure becomes especially effective in the case where, for example, it may be impossible to sufficiently ensure the installation space for the sealing layer 14B in the peripheral area 10B of the sensor substrate 11, in the case where the width of the moisture-proof layer 14a is desired to be set as large as possible, and the like.

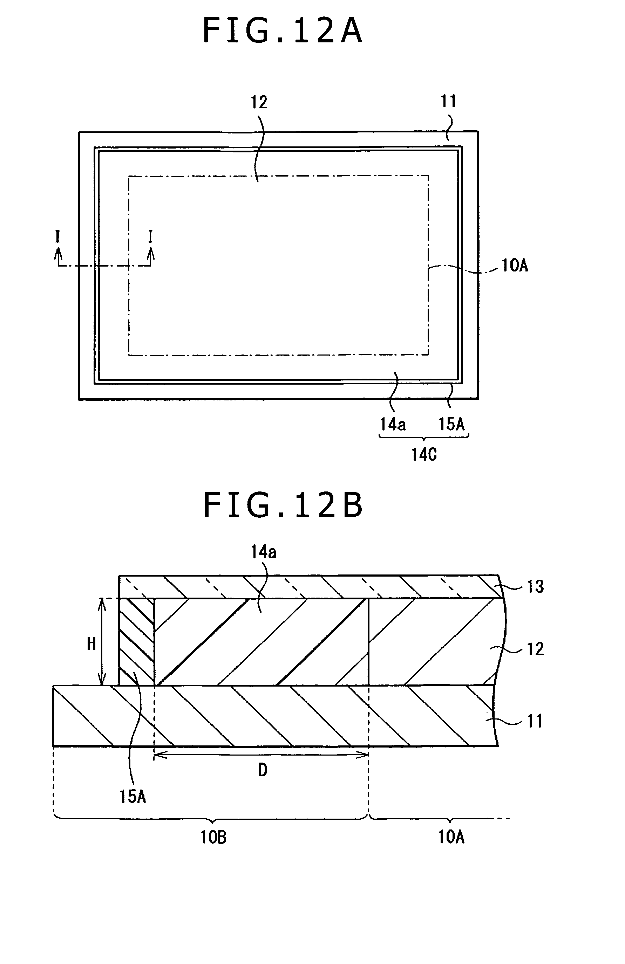

Hereinafter, Changes (Changes 1 to 5) of the radiation imaging device of the first embodiment described above will be described in detail with reference to FIGS. 12A and 12B to FIG. 16. It is noted that constituent elements similar to those in the radiation imaging device 1 according to the first embodiment of the present disclosure are designated by the same reference numerals or symbols, respectively, and a description thereof is omitted here for the sake of simplicity. In addition, in this case, for the sake of convenience, points (a sealing structure of the circumference of the scintillator layer) different from the case of the radiation imaging device 1 of the first embodiment will be mainly described below.

### 3. Change 1

FIG. 12A is a top plan view showing a planar layout of the scintillator layer 12 and a sealing layer 14C which are disposed on the sensor substrate 11. FIG. 12B is a partial cross sectional view of a structure in the vicinity of the boundary between the pixel portion 10A and the peripheral area 10B thereof along line I - I of FIG. 12A. Also, in FIG. 12A, an illustration of the thin plate glass 13 is omitted for the sake of convenience. In Change 1 as well, similarly to the case of the first embodiment described above, the sealing layer 14C with which the circumference of the scintillator layer 12 is sealed includes the moisture-proof layer 14a functioning as the adhesion layer for the thin plate glass 13.

However, in Change 1, the sealing layer 14C is composed of the wall portion 15A and the moisture-proof layer 14a. That is to say, the wall portion 15A is disposed away from the scintillator layer 12, and the moisture-proof layer 14a is provided between the wall portion 15A and the scintillator layer 12. In this Change 1, the adopting of such a structure results in that the thickness H and width D of the moisture-proof layer 14a are controlled in accordance with the height and disposition portion of the wall portion 15A. Specifically, the thickness of the moisture-proof layer 14a is controlled in accordance with the height H of the wall portion 15A, and the width D of the moisture-proof layer 14a is controlled in accordance with the distance from the side surface of the scintillator layer 12 to the wall portion 15A. In the deposition process for such a periphery sealing structure, it is better to form the scintillator layer 12, the wall portion 15A, and the moisture-proof layer 14a in this order.

As with Change 1, one wall portion 15A may be provided in the sealing layer 14C, and the moisture-proof layer 14a may be provided between the wall portion 15A and the scintillator layer 12. Even in this case, in the process for depositing the moisture-proof layer 14a, the wall portion 15A functions as the dam, and the resin material becoming the moisture-proof layer 14a is accumulated in the space defined between the wall portion 15A and the scintillator layer 12. That is to say, the width D and thickness H of the moisture-proof layer 14a are controlled in accordance with the distance between the wall portion 15A and the side surface of the scintillator layer 12, and the like. Therefore, it is possible to obtain the same effects as those of the first embodiment described above.

### 4. Change 2

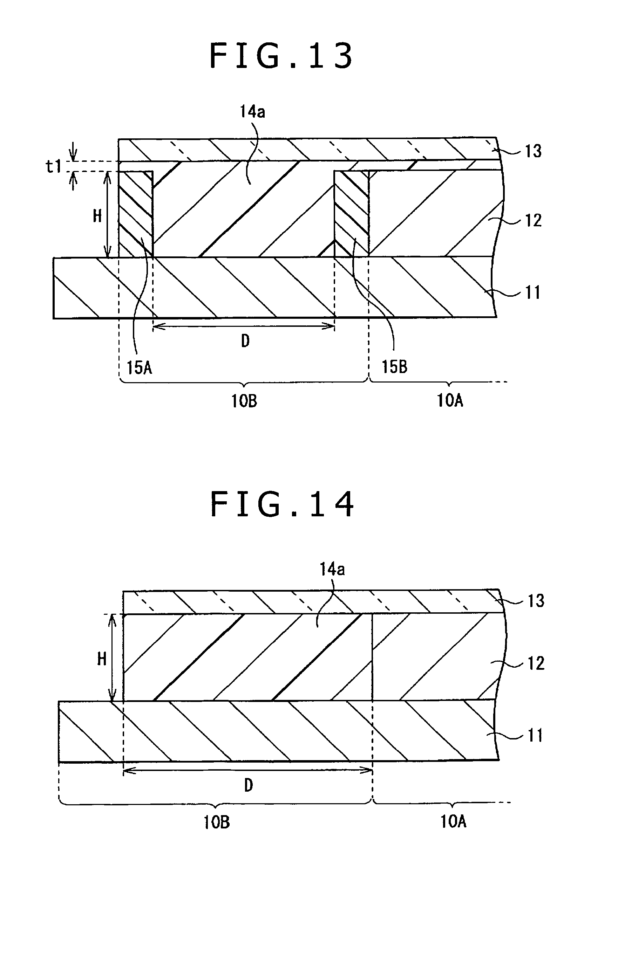

FIG. 13 is a partial cross sectional view of a structure in the vicinity of the boundary between the pixel portion 10A and the peripheral area 10B thereof according to Change 2 of the first embodiment. In Change 2 as well, similarly to the case of the first embodiment described above, the circumference of the scintillator layer 12 is sealed with the moisture-proof layer 14a held between the two wall portions 15A and 15B, and the moisture-proof layer 14a functions as the adhesion layer for the thin plate glass 13.

However, in Change 2, the moisture-proof layer 14a is formed so as to cover not only the circumference of the scintillator layer 12, but also an upper surface of the scintillator layer 12 through a portion thereof having a thickness t1.

The moisture-proof layer 14a may cover not only the circumference of the scintillator layer 12, but also the upper surface of the scintillator layer 12 in such a manner. Even in this case, in the process for depositing the moisture-proof layer 14a, the wall portions 15A and 15B function as the dam. Also, a most part of the resin material becoming the moisture-proof layer 14a is accumulated in the space defined between the two wall portions 15A and 15B, and a part thereof is adhered to the upper surface of the wall portions 15A and 15B. That is to say, the width D and thickness H of the moisture-proof layer 14a are mainly controlled in accordance with the heights, the disposition interval, and the like of the wall portions 15A and 15B. Therefore, it is possible to obtain the effects comparable to those of the first embodiment described above. In addition, in Change 2, the moisture-proof layer 14a having the adhesion property and the moisture-proof property is also formed between the scintillator layer 12 and the thin plate glass 13. As a result, the adhesiveness and the moisture-proof property for the thin plate glass 13 are improved, and thus it is possible to more effectively prevent the moisture from penetrating into the scintillator layer 12.

### 5. Change 3

FIG. 14 is a partial cross sectional view of a structure in the vicinity of the boundary between the pixel portion 10A and the peripheral area 10B thereof according to Change 3 of the first embodiment. Similarly to the case of the first embodiment described above, Change 3 also has the structure in which the scintillator layer 12 is hermetically sealed with both of the moisture-proof layer 14a and the thin plate glass 13. In addition, a thickness of the thin plate glass 13 is set equal to or smaller than 0.1 mm. As a result, as previously stated, the thin plate glass 13 has the resistance property against the bending stress as well generated while the thin plate glass 13 is stuck to the sensor substrate 11, and thus can also follow the deformation or the like caused in the phase of the curing of the moisture-proof layer 14a.

### 6. Change 4

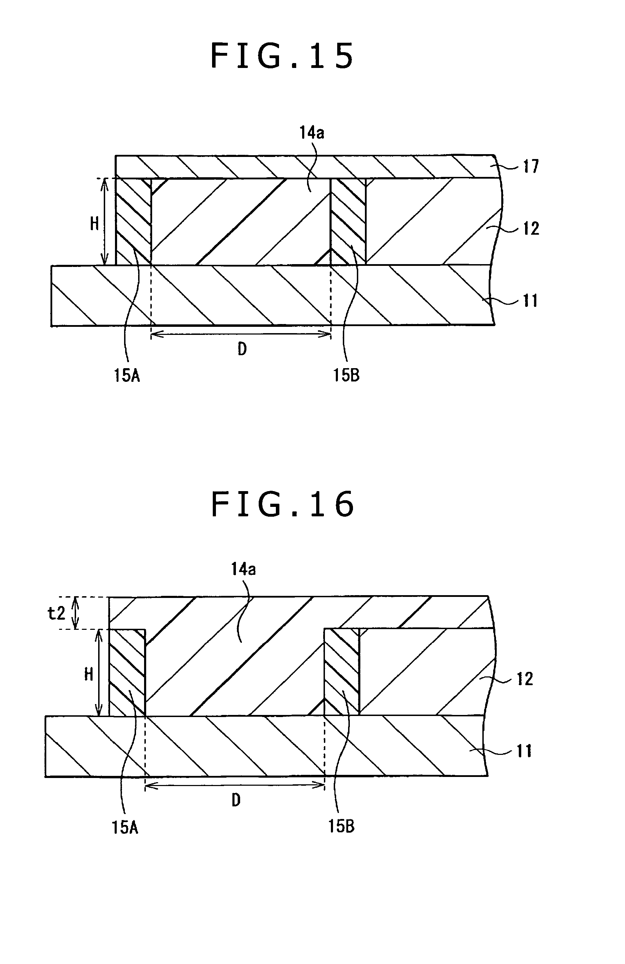

FIG. 15 is a partial cross sectional view of a structure in the vicinity of the boundary between the pixel portion 10A and the peripheral area 10B thereof according to Change 4 of the first embodiment. In Change 4 as well, similarly to the case of the first embodiment described above, the circumference of the scintillator layer 12 is sealed with the moisture-proof layer 14a held between the two wall portions 15A and 15B. Also, the moisture-proof layer 14a functions as the adhesion layer for a sealing plate with which the upper surface of the scintillator layer 12 is sealed. However, Change 4 adopts a structure in which a thin plate metallic member 17 is used as the sealing plate instead of using the thin plate glass 13. The thin plate metallic member 17 is made of a metal which transmits the radiation, for example, a simple substrate of beryllium (Be), aluminum (Al), a silver (Ag), titanium (Ti), and nickel (Ni), or an alloy containing therein any of these metals. Or, the thin plate metallic member 17 may also be a plate material, made of a light metal, such as a carbon plate.

As has been described, the sealing plate which is stuck to the upper surface of the scintillator layer 12 is by no means limited to the thin plate glass 13 described in the first and second embodiments described above, and thus the metallic plate may also be used as that sealing plate. Even in this case, the circumference of the scintillator layer 12 is sealed with the moisture-proof layer 14a held, as the adhesion layer for the thin plate metallic member 17, between the two wall portions 15A and 15B, whereby it is possible to obtain the effects comparable to those of the first embodiment described above. However, the case of using the thin plate glass 13 results in that the difference in coefficient of linear expansion between the sensor substrate 11 and the thin plate glass 13 can be reduced as described above, and the UV is easy to transmit as compared with the case of using the thin plate metallic member 17. For this reason, the UV curable resin can be used as the adhesive agent during the sticking instead of using the thermosetting resin as the adhesive agent. Therefore, it is possible to reduce the thermal stress in the phase of the sticking, and it is also possible to suppress the generation of the warpage of the panel. In addition thereto, the using of the thin plate glass 13 is excellent in weight saving and is also satisfactory in the X-ray transmission rate.

### 7. Change 5

FIG. 16 is a partial cross sectional view of a structure in the vicinity of the boundary between the pixel portion 10A and the peripheral area 10B thereof according to Change 5 of the first embodiment. In Change 5 as well, similarly to the case of the first embodiment described above, the circumference of the scintillator layer 12 is sealed with the moisture-proof layer 14a held between the two wall portions 15A and 15B. However, in Change 5, neither the thin plate glass 13 in the first embodiment described above, nor the thin plate metallic member 17 in Change 4 described above is stuck as the sealing plate to the sensor substrate 11 through the scintillator layer 12. Instead, the moisture-proof layer 14a is formed so as to cover the upper surface as well of the scintillator layer 12 through a portion thereof having a thickness t2. The thickness t2 is preferably set to a thickness enough to suppress the penetration of the moisture into the scintillator layer 12. In Change 5, the scintillator layer 12 is hermetically sealed only with the moisture-proof layer 14a in such a manner.

As with Change 5, the sealing plate made of the glass or the like may not be stuck onto the scintillator layer 12, and thus the scintillator layer 12 may also be covered with the resin having the moisture-proof property. Even in such a case, the moisture-proof layer 14a is held between the two wall portions 15A and 15B in the circumference of the scintillator layer 12, whereby it is possible to obtain the effects comparable to those of the first embodiment described above.

### 8. Example of Application

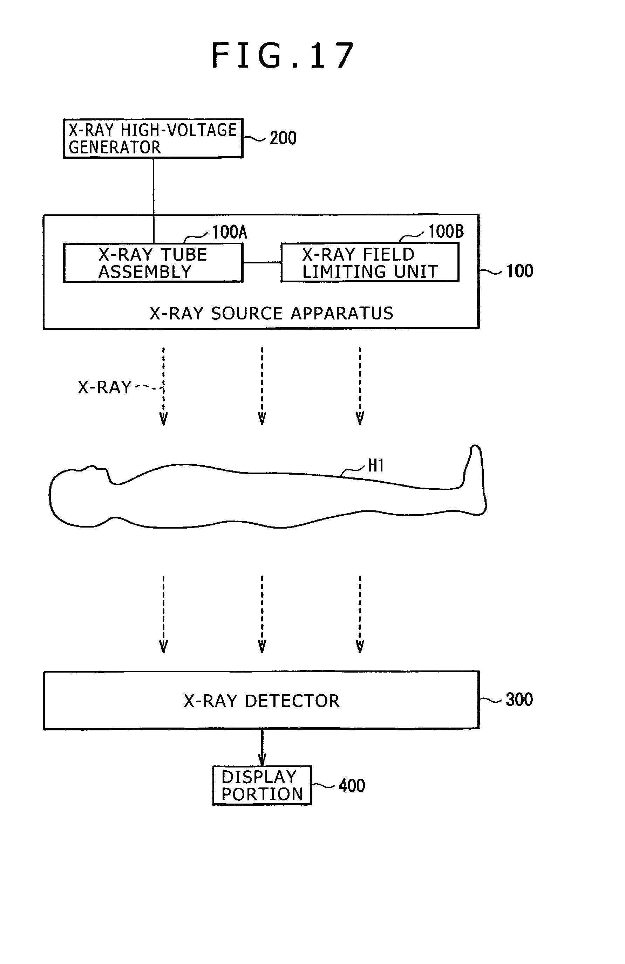

FIG. 17 shows a configuration of a radiation imaging display system to which the radiation imaging device described in the first embodiment is applied. This radiation imaging display system acquires image data based on an X-ray (transmission intensity) transmitted through a test body H1, and displays the image data in the form of an image. This radiation imaging display system, for example, is composed of an X-ray source apparatus 100, an X-ray high-voltage cable with a plug (not shown), an X-ray high-voltage generator 200, an X-ray detector 300 (corresponding to the radiation imaging device of the first embodiment), and a display portion 400.

In this case, the X-ray high-voltage cable with a plug guides a high voltage into an X-ray tube assembly 100A, and the X-ray high-voltage generator 200 generates the high voltage. Also, the display portion 400 displays the image data in the form of an image on a two-dimensional plane. The X-ray source apparatus 100, for example, includes an X-ray tube assembly 100A necessary to generate the X-ray, and an X-ray field limiting unit 100B for limiting a range of the X-ray generated.

In the radiation imaging display system, the X-ray generated from the X-ray source apparatus 100 is irradiated to the test body H1, transmitted through the test body H1, and is detected by the X-ray detector 300, whereby the image based on the intensity distribution of the transmitted X-ray is displayed on the display portion 400.

Although the present disclosure has been described so far based on the embodiments and Changes, the present disclosure is by no means limited thereto, and thus various changes can be made. For example, a predetermined reflecting metallic film may be provided between the scintillator layer 12 and the thin plate glass 13. As a result, the fluorescence emitted upward (to the thin plate glass 13 side) from the scintillator layer 12 is reflected to the sensor substrate 11 side, thereby making it possible to increase a quantity of received light in the pixel portion 10A. In addition, the provision of the reflecting metallic film results in improvement in the moisture-proof property.

In addition, in the embodiments and Changes, the case where the plate member (sealing plate) stuck onto the scintillator layer is composed of either the glass (thin plate glass) or the metallic member (thin plate metallic member) has been exemplified. However, the material for the sealing plate is by no means limited to the glass or the metallic material, and thus may also be made of any other suitable inorganic material or organic material.

The present disclosure contains subject matter related to that disclosed in Japanese Priority Patent Application JP 2011-050154 filed in the Japan Patent Office on March 8, 2011, the entire content of which is hereby incorporated by reference.

It should be understood by those skilled in the art that various modifications, combinations, sub-combinations and alterations may occur depending on design requirements and other factors insofar as they are within the scope of the appended claims or the equivalents thereof.

Support for the claims and further embodiments are defined in the following itemized list:
1. A radiation imaging device, comprising:
   a sensor substrate having a pixel portion including a photoelectric conversion element;
   a scintillator layer provided on said pixel portion of said sensor substrate; and
   a sealing layer with which at least a part of said scintillator layer is sealed,
   wherein said sealing layer includes
   a first wall portion disposed on said sensor substrate away from said scintillator layer, and
   a moisture-proof layer provided between said scintillator layer and said first wall portion.
2. The radiation imaging device according to item 1, wherein a second wall portion is provided on a scintillator layer side of said first wall portion, and said moisture-proof layer is held between said first and second wall portions.
3. The radiation imaging device according to item 2, wherein when let H and D be a thickness and a width of said moisture-proof layer, respectively, an aspect ratio (H/D) of the thickness H to the width D is equal to or larger than 0.6.
4. The radiation imaging device according to item 3, wherein each of said first and second wall portions is formed by laminating plural resin layers made of identical or different resin materials one upon another.
5. The radiation imaging device according to item 2, wherein said first and second wall portions are disposed so as to surround said scintillator layer on said sensor substrate.
6. The radiation imaging device according to item 5, wherein a sealing plate is provided on said sensor substrate.
7. The radiation imaging device according to item 6, wherein said moisture-proof layer is made of a resin having a adherence property, and said sealing plate is stuck to said scintillator layer through said moisture-proof layer.
8. The radiation imaging device according to item 6, wherein a thickness of said sealing plate is equal to or smaller than 0.1 mm.
9. The radiation imaging device according to item 8, wherein said sealing plate is made of a glass.
10. A method of manufacturing a radiation imaging device, comprising:
   forming a scintillator layer on a pixel portion of a sensor substrate having said pixel portion including a photoelectric conversion element; and
   forming a sealing layer in a circumference area of said pixel portion on said sensor substrate,
   wherein in forming said sealing layer, after a first wall portion is formed in said circumference area, a moisture-proof layer is formed in an area on the pixel portion side of said first wall portion.
11. The method of manufacturing a radiation imaging device according to item 10, wherein in forming said sealing layer, a second wall portion is formed on a scintillator layer side of said first wall portion, and said moisture-proof layer is applied between said first and second wall portions.
12. The method of manufacturing a radiation imaging device according to item 11, wherein heights and a disposition interval of said first and second wall portions are designed in such a way that when let H and D be a thickness and a width of said moisture-proof layer, respectively, an aspect ratio (H/D) of the thickness H to the width D becomes equal to or larger than 0.6.
13. The method of manufacturing a radiation imaging device according to item 12, wherein each of said first and second wall portions is formed by laminating resin materials identical to or different from one another one upon another.
14. The method of manufacturing a radiation imaging device according to item 11, wherein said first and second wall portions are disposed so as to surround said scintillator layer in said circumference area on said sensor substrate.
15. The method of manufacturing a radiation imaging device according to item 14, further comprising:
   sticking a sealing plate onto said scintillator layer.
16. The method of manufacturing a radiation imaging device according to item 15, wherein said moisture-proof layer is made of a resin having an adherence property, and said sealing plate is stuck onto said scintillator layer through said moisture-proof layer.
17. The method of manufacturing a radiation imaging device according to item 15, wherein a thickness of said sealing plate is set equal to or smaller than 0.1 mm.
18. The method of manufacturing a radiation imaging device according to item 17, wherein a glass is used as said sealing plate.

## Claims

1. A radiation imaging device, comprising:
a sensor substrate having a pixel portion including a photoelectric conversion element;
a scintillator layer provided on said pixel portion of said sensor substrate; and
a sealing layer with which at least a part of said scintillator layer is sealed,
wherein said sealing layer includes
a first wall portion disposed on said sensor substrate away from said scintillator layer, and
a moisture-proof layer provided between said scintillator layer and said first wall portion.

2. The radiation imaging device according to claim 1, wherein a second wall portion is provided on a scintillator layer side of said first wall portion, and said moisture-proof layer is held between said first and second wall portions.

3. The radiation imaging device according to claim 2, wherein when let H and D be a thickness and a width of said moisture-proof layer, respectively, an aspect ratio (H/D) of the thickness H to the width D is equal to or larger than 0.6.

4. The radiation imaging device according to claim 3, wherein each of said first and second wall portions is formed by laminating plural resin layers made of identical or different resin materials one upon another.

5. The radiation imaging device according to any of claims 2 to 4, wherein said first and second wall portions are disposed so as to surround said scintillator layer on said sensor substrate.

6. The radiation imaging device according to claim 5, wherein a sealing plate is provided on said scintillator layer.

7. The radiation imaging device according to claim 6, wherein said moisture-proof layer is made of a resin having a adherence property, and said sealing plate is stuck to said scintillator layer through said moisture-proof layer.

8. The radiation imaging device according to claim 6, wherein a thickness of said sealing plate is equal to or smaller than 0.1 mm.

9. The radiation imaging device according to claim 8, wherein said sealing plate is made of a glass.

10. A method of manufacturing a radiation imaging device, comprising:
forming a scintillator layer on a pixel portion of a sensor substrate having said pixel portion including a photoelectric conversion element; and
forming a sealing layer in a circumference area of said pixel portion on said sensor substrate,
wherein in forming said sealing layer, after a first wall portion is formed in said circumference area, a moisture-proof layer is formed in an area on the pixel portion side of said first wall portion.

11. The method of manufacturing a radiation imaging device according to claim 10, wherein in forming said sealing layer, a second wall portion is formed on a scintillator layer side of said first wall portion, and said moisture-proof layer is applied between said first and second wall portions.

12. The method of manufacturing a radiation imaging device according to claim 11, wherein heights and a disposition interval of said first and second wall portions are designed in such a way that when let H and D be a thickness and a width of said moisture-proof layer, respectively, an aspect ratio (H/D) of the thickness H to the width D becomes equal to or larger than 0.6.

13. The method of manufacturing a radiation imaging device according to claim 12, wherein each of said first and second wall portions is formed by laminating resin materials identical to or different from one another one upon another.

14. The method of manufacturing a radiation imaging device according to any of claims 11 to 13, wherein said first and second wall portions are disposed so as to surround said scintillator layer in said circumference area on said sensor substrate.

15. The method of manufacturing a radiation imaging device according to claim 14, further comprising:
sticking a sealing plate onto said scintillator layer.
